# EUROPÄISCHE PATENTSCHRIFT

(11) **EP 4 076 065 B1**
(45) Veröffentlichungstag und Bekanntmachung des Hinweises auf die Patenterteilung: **25.10.2023**
(21) Anmeldenummer: 20839272.0
(22) Anmeldetag: 17.12.2020
(51) Int. Cl.: A24F 40/46, A24F 40/70

(54) **VERDAMPFEREINHEIT, VERDAMPFERBAUGRUPPE SOWIE VERDAMPFERKARTUSCHE FÜR EINEN INHALATOR, INHALATOR MIT EINER SOLCHEN VERDAMPFERKARTUSCHE SOWIE VERFAHREN ZUR HERSTELLUNG VON VERDAMPFEREINHEITEN**
VAPORIZER UNIT, VAPORIZER ASSEMBLY AS WELL AS VAPORIZER CARTRIDGE FOR AN INHALER, INHALER WITH SUCH A VAPORIZER CARTRIDGE AS WELL AS METHOD FOR PRODUCING VAPORIZER UNITS
ENSEMBLE DE VAPORISATION, MODULE DE VAPORISATION ET CARTOUCHE DE VAPORISATION POUR INHALATEUR, INHALATEUR DOTÉ D'UNE TELLE CARTOUCHE DE VAPORISATION ET PROCÉDÉ DE FABRICATION D'ENSEMBLES DE VAPORISATION

(30) Priorität: 19.12.2019 DE 102019135177
(43) Veröffentlichungstag der Anmeldung: 26.10.2022
(73) Patentinhaber: Körber Technologies GmbH, 21033 Hamburg (DE)
(72) Erfinder: CORNILS, Lasse, 22605 Hamburg (DE); HANNEKEN, Christian, 22299 Hamburg (DE); KÄTHNER, Svenja, 20259 Hamburg (DE); KLEINE WÄCHTER, Michael, 23881 Lankau (DE); ROMMING, Niklas, 24306 Plön (DE); SCHUSTER, Christof, 20259 Hamburg (DE); ROSENBOHM, Arne, 21720 Steinkirchen (DE); NIEBUHR, Gunnar, 21149 Hamburg (DE); SCHMIDT, Rene, 21244 Buchholz i.d.N. (DE); PANKOKE, Finn Oliver, 25436 Uetersen (DE); LEICHSENRING, Dennis, 22767 Hamburg (DE); JAKLIN, Jan, 70736 Fellbach (DE); BERGMANN, Max, 22303 Hamburg (DE)
(74) Vertreter: Stork Bamberger Patentanwälte PartmbB
(86) Internationale Anmeldenummer: PCT/EP2020/086694
(87) Internationale Veröffentlichungsnummer: WO 2021/122936

(56) Entgegenhaltungen:
- WO-A1-2018/219584
- WO-A1-2019/127643
- DE-A1-102017 130 501
- DE-A1-102018 105 220
- US-A1- 2018 116 285

## Beschreibung

Die Erfindung betrifft ein Verfahren zum Herstellen von Verdampfereinheiten als Bestandteil von Inhalatoren, umfassend die Schritte: a) Bereitstellen eines flexiblen Leiterkartenmaterials mit einer Vielzahl von Bauplätzen für einzelne Verdampfereinheiten, wobei das flexible Leiterkartenmaterial mindestens mit hinsichtlich Position und/oder Verlauf vorbestimmten Leiterbahnen und/oder Vorstanzungen für jeden Bauplatz wahlweise vorstrukturiert ist oder vorstrukturiert wird, b) Bereitstellen und Platzieren mindestens eines elektrisch mit den Leiterbahnen wahlweise verbundenen oder verbindbaren Heizorgans an dem oder jedem Bauplatz, und c) mindestens teilweises Abdecken jedes Bauplatzes mit einem Dichtmaterial zur Bildung einer partiellen Ummantelung für jede gebildete Verdampfereinheit.

Des Weiteren betrifft die Erfindung Verdampfereinheiten, Verdampferbaugruppen und Verdampferkartuschen als Bestandteil von Inhalatoren.

Die Erfindung betrifft auch Inhalatoren, ausgebildet und eingerichtet zum Inhalieren von mit Wirkstoffen und/oder Aromastoffen angereichertem Dampf/Aerosol.

Solche Verdampfereinheiten, Verdampferbaugruppen, Verdampferkartuschen und Inhalatoren kommen in der Genussmittelindustrie, hier insbesondere im Zusammenhang mit einer elektronischen Zigarette, der so genannten E-Zigarette, sowie im medizinischen Bereich zum Einsatz, um fluide Genussmittel und/oder fluide medizinische Produkte in Dampfform und/oder als Aerosole inhalieren zu können. Beim Konsumieren saugt üblicherweise eine Person an einem Mundstück des Inhalators, wodurch in einem Luftströmungskanal ein Saugdruck entsteht, der einen Luftstrom durch den Luftströmungskanal erzeugt. Der Luftstrom kann aber auch maschinell z.B. durch eine Pumpe erzeugt werden. In dem Luftströmungskanal wird dem Luftstrom eine von der Verdampfereinheit erzeugte und bereitgestellte verdampfte Flüssigkeit zugegeben, um der konsumierenden Person ein Aerosol oder ein Aerosol-Dampf-Gemisch zu verabreichen. Die Flüssigkeit ist an der oder in der Verdampferkartusche bevorratet. Als Flüssigkeit kommen unterschiedliche Mischungen mit verschiedenen Bestandteilen gleicher oder unterschiedlicher Dampfdichten zum Einsatz. Eine typische Mischung für den Einsatz in einer E-Zigarette weist z.B. Bestandteile von Glycerin und Propylenglycol auf, ggf. angereichert um Nikotin und/oder nahezu beliebige Geschmacks- und/oder Aromastoffe. Für den Einsatz im medizinischen oder therapeutischen Bereich, z.B. zur Inhalation von Asthma-Präparaten, kann die Mischung entsprechend medizinische Bestandteile und Wirkstoffe aufweisen.

Eine zentrale Bedeutung in dem Inhalator kommt der Verdampfereinheit zu, die üblicherweise ein Einwegartikel oder Bestandteil eines Einwegartikels ist. Es ist ein zunehmender Trend für den Einsatz solcher Verdampfereinheiten zu erkennen, der dazu führt, dass die Verdampfereinheiten, Verdampferbaugruppen und Verdampferkartuschen und Inhalatoren in sehr großen Stückzahlen zu produzieren sind, insbesondere dann, wenn die Verdampferbaugruppen und/oder Verdampferkartuschen mit den Verdampfereinheiten als Einwegartikel ausgebildet sind. Mit anderen Worten handelt es sich bei den Verdampfereinheiten um ein Massenprodukt. Die bisher bekannten Verfahren zur Herstellung von Verdampfereinheiten sind nur bedingt geeignet, große Stückzahlen schnell und effizient herzustellen, um diese dann massenhaft schnell und effizient in Verdampferbaugruppen und Verdampferkartuschen montieren zu können bzw. daraus Inhalatoren herstellen zu können.

Die Verdampfereinheit ist Bestandteil der Verdampferbaugruppe. Die Verdampferbaugruppe ist Bestandteil der Verdampferkartusche. Die Verdampferkartusche bildet zusammen mit einem Kartuschenträger und einem Mundstück den Inhalator. Die einzelnen Bestandteile der Verdampferkartusche, nämlich mindestens ein Hohlkörper, ein Vorratstank und die Verdampfereinheit, können in einem gemeinsamen Bauteil zusammengefasst sein, wobei dieses Bauteil dann ein Einwegartikel ist, der für eine endliche Anzahl von Inhalationszügen durch eine konsumierende Person ausgelegt ist und zusammen mit einem Kartuschenträger als wiederverwendbarem Mehrwegartikel, der mindestens eine elektronische Steuereinheit und eine Energiequelle umfasst, einen Inhalator bildet. Die Verdampferkartusche kann jedoch auch erst durch das Zusammenfügen mehrerer Bauteile gebildet sein, wobei einzelne Bauteile, nämlich insbesondere der Hohlkörper und die Verdampfereinheit, in dem Kartuschenträger als Mehrwegartikel angeordnet sind, und der Vorratstank als separates Bauteil den Einwegartikel bildet. Letztlich lässt sich der Inhalator durch Austausch des Einwegartikels, der üblicherweise die Flüssigkeit beinhaltet, variabel einsetzen.

Entsprechend sind der Einwegartikel und der Mehrwegartikel lösbar miteinander verbunden. Der Kartuschenträger als Mehrwegartikel umfasst üblicherweise mindestens eine elektronische Steuereinheit und eine elektrische Energiequelle. Die Energiequelle kann z.B. eine elektrochemische Einwegbatterie oder ein wiederaufladbarer elektrochemischer Akku, z.B. ein Lithium-Ionen- oder Lithium-Polymer-Akku sein, mittels dem ein Heizorgan über elektrische Kontakte der Verdampfereinheit mit Energie versorgt wird. Die elektronische und/oder elektrische Steuereinheit dient zum Steuern der Verdampfereinheit innerhalb der Verdampferkartusche. Der Kartuschenträger kann aber auch Bestandteile der Verdampferkartusche umfassen. Der Einwegartikel kann als Ansteckteil an den Mehrwegartikel ansteckbar oder als Einsetzteil in den Mehrwegartikel einsetzbar ausgebildet sein. Anstelle einer Steckverbindung sind auch Schraubverbindungen, Schnappverbindungen oder andere Schnellverbindungen einsetzbar. Mit der Verbindung von Einwegartikel und Mehrwegartikel wird eine mechanische und elektrische Kopplung zur Bildung eines funktionsbereiten Inhalators hergestellt.

Eine letztlich die Nutzung (z.B. als E-Zigarette oder als medizinischer Inhalator) bestimmende Komponente ist der Vorratstank als Bestandteil der Verdampferkartusche. Diese beinhaltet in der Regel die von der Person gewählte, gewünschte und/oder benötigte Flüssigkeit bzw. ein Flüssigkeitsgemisch (im Folgenden auch allgemein als Fluid bezeichnet) sowie den den Luftströmungskanal bildenden Hohlkörper und die Verdampfereinheit. Das Fluid ist in dem Vorratstank der Verdampferkartusche bevorratet. Mittels der - bedingt durch Mikrokanäle - flüssigkeitspermeablen Verdampfereinheit wird das Fluid aus dem Vorratstank aufgrund zumindest initial kapillarer Förderung durch ein Dochtorgan und das Heizorgan geleitet. Die von einer Energiequelle erzeugte Spannung, die an dem Heizorgan angelegt wird, führt zu einem Stromfluss im Heizorgan. Aufgrund des Heizwiderstandes, vorzugsweise des Ohm'schen Widerstands des Heizorgans führt der Stromfluss zu einer Erhitzung des Heizorgans und letztlich zu einer Verdampfung des in der Verdampfereinheit befindlichen Fluids. Der auf diese Weise erzeugte Dampf und/oder Aerosol entweicht aus der Verdampfereinheit in Richtung des Luftströmungskanals und wird als Dampfzugabe der Luftströmung beigemischt. Das Fluid hat damit einen vorgegebenen Weg mit einer vorgegebenen Strömungsrichtung, nämlich als Fluid durch das Dochtorgan an das und durch das Heizorgan und gasförmig aus dem Heizorgan in den Luftströmungskanal. In dem Luftströmungskanal wird das verdampfte Fluid durch den Luftstrom mitgerissen, wobei sich Dampf/Nebel und/oder Aerosol bildet, wenn der Luftströmungskanal mit einem Druck/Unterdruck beaufschlagt wird, indem z.B. eine konsumierende Person an dem Luftströmungskanal saugt oder eine Pumpe einen Luftstrom durch den Luftströmungskanal fördert.

Damit das Fluid aus dem Vorratstank nicht direkt in den Luftströmungskanal fließt, deckt die Verdampfereinheit den Zugang vom Vorratstank zum Luftströmungskanal vollständig ab. Vollständig abgedeckt bedeutet in diesem Zusammenhang, dass die Flüssigkeit zwingend durch die Verdampfereinheit geführt ist, so dass das Fluid nicht direkt vom Vorratstank in den Luftströmungskanal gelangen kann, sondern den "Umweg" über das Dochtorgan und das Heizorgan nehmen muss. Das Dochtorgan dient zum einen zum Zwischenspeichern von Fluid, um insbesondere bei nahezu entleertem Vorratstank noch ausreichend Fluid für wenige Züge am Inhalator zur Verfügung zu stellen. Das Dochtorgan dient zum anderen insbesondere zum Transport des Fluids vom Vorratstank in Richtung des Luftströmungskanals und wirkt gleichzeitig als eine Art Rückschlagschutz, um den Rücklauf von Fluid und/oder Gas bzw. Dampf in Richtung des Vorratstanks zu unterbinden sowie eine Anreicherung einzelner Bestandteile des Fluids bei höheren Temperaturen zu verhindern.

Bekannte Verfahren zur Herstellung von Verdampfereinheiten sehen vor, dass Heizorgane, z.B. im Wesentlichen Silizium oder dotiertes Silizium aufweisende Bauteile, mittels Direktkontaktierung elektrisch mit Leiterbahnen auf einem Keramiksubstrat verbunden werden, derart, dass das Heizorgan eine in der Keramik befindliche Durchlassöffnung abdeckt. Diese Verfahrensweise weist nur eine bedingte Tauglichkeit für die Massenfertigung auf. Derartig hergestellte Verdampfereinheiten sind einerseits im Hinblick auf die Dichtigkeit - mit Ausnahme der Mikrokanäle im Heizorgan - der Durchlassöffnung, und andererseits bei der Montage der Verdampfereinheit mit die Verdampfereinheit umgebenden Komponenten des Inhalators problematisch. Um sicherzustellen, dass weder Flüssigkeit in den Luftströmungskanal gelangt noch Flüssigkeit aus der Verdampferkartusche bzw. dem Inhalator ausläuft, sind zusätzliche Dichtmittel z.B. in Form von Dichtringen oder dergleichen notwendig. Dies führt zu einer Vielzahl von Einzelteilen mit entsprechend engen Toleranzen, so dass die Montage der mit den bekannten Verfahren hergestellten Verdampfereinheiten anspruchsvoll und teuer ist, zumal die Automatisierung der Herstellung teilweise sehr komplex und daher sehr aufwändig und technisch schwierig ist.

In weiteren bekannten Verfahren zur Herstellung von Verdampfereinheiten werden mit Kunststoff umspritzte metallische Stanzbänder mit Heizorganen bestückt. Die Heizorgane werden vorfixiert und dann erfolgt eine elektrische Kontaktierung der Heizorgane mit Leiterbahnen des Stanzbandes z.B. mittels Wirebonding. Diese Verfahrensweise ist zum einen aufwändig und damit teuer, da eine automatisierte Schrittfolge mit standardisierten Prozessen nur in angepassten Vorrichtungen/Maschinen/Automaten ausgeführt werden kann. Des Weiteren besteht auch bei den so gefertigten Verdampfereinheiten die Dichtproblematik, da durch die Teilevielfalt insbesondere beim Montieren der Verdampfereinheiten Komponenten des Inhalators, die die Verdampfereinheit umgeben, mehrere Komponenten gegeneinander mit zusätzlichen Dichtmitteln abgedichtet werden müssen. Dadurch ist die Montage der mit den bekannten Verfahren hergestellten Verdampfereinheiten anspruchsvoll und teuer.

Ein Verfahren mit den Schritten des Oberbegriffes des Anspruches 1 sowie eine Verdampfereinheit mit den Merkmalen des Oberbegriffes des Anspruches 24 sind aus der DE 10 2017 130501 A1 bekannt.

Der Erfindung liegt somit die Aufgabe zugrunde, ein effizientes Verfahren zur massenhaften Herstellung von montagefreundlichen und hinsichtlich Dichteigenschaften optimierten Verdampfereinheiten vorzuschlagen. Die Aufgabe besteht weiterhin darin, montagefreundliche Verdampfereinheiten, Verdampferbaugruppen, Verdampferkartuschen und Inhalatoren zu schaffen, die automatisiert und effizient als Massenartikel herstellbar sind und optimierte Dichteigenschaften aufweisen.

Diese Aufgabe wird gemäß einem ersten Aspekt der Erfindung insbesondere durch das eingangs genannte Verfahren dadurch gelöst, dass das Dichtmaterial derart aufgebracht wird, dass die aus Dichtmaterial gebildete Ummantelung jedes Heizorgan unter Freihaltung einer Heizfläche mindestens auf einer Oberseite des flexiblen Leiterkartenmaterials mindestens in einem Randbereich abdeckt, und mindestens die vom Leiterkartenmaterial und/oder dem Heizorgan wegweisenden Außenflächen der Ummantelung Dichtflächen bilden. Mit dieser Schrittfolge ist die Herstellung von Verdampfereinheiten als Massenartikel ohne zusätzliche Entwicklung von Sondermaschinen oder dergleichen gewährleistet. Die Schrittfolge kann vor allem von der Anlieferung der Einzelteile bis zur einbaufertigen Verdampfereinheit innerhalb einer aus bekannten Vorrichtungen/Maschinen zusammengestellten Fertigungslinie mindestens teilautomatisiert, vorzugsweise jedoch vollautomatisiert ausgeführt werden, wodurch die Logistikanforderung bei der Herstellung lediglich auf die Zufuhr der Einzelteile, wie flexibles Leiterkartenmaterial und Heizorgane und Dichtmaterial, und den Abtransport der hergestellten Verdampfereinheiten kostensparend beschränkt ist.

Das flexible Leiterkartenmaterial ist ein günstiges Trägermaterial und ermöglicht eine energiesparende Ausführung der Leiterbahnen mit einem nahezu beliebig auslegbaren Leiterquerschnitt. Insbesondere können möglichst dünne und möglichst schmale Leiterbahnquerschnitte für eine optimierte Energieeffizienz bereitgestellt werden. Die Verwendung des flexiblen Leiterkartenmaterials und dessen Bereitstellung ermöglichen gegenüber dem Bereitstellen von Keramik oder dem mit Kunststoff ummantelten Stanzgitter als Trägermaterial auch kleine Konturen für Leiterbahnen und/oder Vorstanzungen und stellen, frei von prozessbedingten Restriktionen, wie sie beispielsweise beim Stanzgitter-Prozess bestehen, vielfältige Kontaktierungsmöglichkeiten der Leiterbahnen bereit. Beispielsweise kann das Leiterkartenmaterial Polyimid in Form einer Folie aufweisen oder daraus bestehen.

Vorstanzungen im Sinne der Erfindung sind Löcher, Spalte, Freischnitte, Durchbrüche oder dergleichen in dem Leiterkartenmaterial, die die Oberseite mit der Unterseite verbinden. Beispiele für Vorstanzungen sind z.B. umlaufende und nur durch Verbindungsstege unterbrochene Trennspalte oder Durchlassöffnungen bildende Öffnungen an jedem Bauplatz. Als Bauplatz wird ein die Basis für eine herzustellende Verdampfereinheit bildender Anbindungsplatz bezeichnet. Ein Anbindungsplatz ist ein Bereich auf dem Leiterkartenmaterial, der nach der Fertigstellung vom Rest des Leiterkartenmaterials trennbar/vereinzelbar ist und dadurch eine einzelne Verdampfereinheit bildet. Die mehreren Bauplätze auf einer Rolle sind vorzugsweise identisch ausgebildet und bilden so genannte Endlosstränge/Gurte. Auf einem solchen Gurt können die Bauplätze einzeln hintereinander und/oder in zwei oder mehr Reihen nebeneinander, gleichmäßig oder versetzt zueinander oder anderweitig angeordnet/ausgebildet sein. Beispielsweise können die Bauplätze in einem schachbrettartigen Muster oder in einem hexagonalen Muster angeordnet sein. Die elektrischen Leiterbahnen und Vorstanzungen sind für jeden Bauplatz vordefiniert und vorstrukturiert, und zwar in Abhängigkeit der Konfiguration der jeweils zu fertigenden Verdampfereinheiten. Mit anderen Worten ist das flexible Leiterkartenmaterial vorkonfektioniert. Die Vorkonfektionierung kann auch die Heizorgane umfassen, indem an jedem Bauplatz anstelle einer Vorstanzung als Durchlassöffnung für ein separat zu platzierendes Heizorgan in dem Leiterkartenmaterial ein Heizorgan integriert ist, das elektrisch mit den Leiterbahnen verbunden ist. Für diesen Fall fallen die Schritte a) und b) zusammen, da mit der Bereitstellung des flexiblen Leiterkartenmaterials gleichzeitig auch die Heizorgane bereitgestellt werden. Die Verbindung von Heizorgan zu Leiterbahn kann bereits bestehen, z.B. im Falle eines in das Leiterkartenmaterial integrierten Heizorgans als Folienheizer. Ein solches Heizorgan kann flüssigkeitspermeabel sein oder z.B. durch eine Mikrostrukturierung flüssigkeitspermeabel werden. Die Verbindung kann aber auch erst gebildet werden, z.B. in dem Fall, dass das Heizorgan auf den Leiterbahnen platziert wird. Ein solches Heizorgan kann vorteilhafterweise bereits Mikrokanäle aufweisen.

Alternativ oder zusätzlich können die elektrischen Leiterbahnen vor dem Bereitstellen und Platzieren des Heizorgans auf das Leiterkartenmaterial aufgebracht werden. Dies kann beispielsweise mit einem dem Farbdruck ähnlichem Prozess, z.B. Polyjet, Siebdruck oder Stempeldruck, oder einer gezielten Bedampfung stattfinden. Es ist aber auch jeder andere geeignete und bekannte Prozess denkbar. Mit anderen Worten wird das Leiterkartenmaterial durch diesen Schritt vorkonfektioniert bzw. vorstrukturiert. Alternativ oder zusätzlich können Vorstanzungen auch vor oder nach dem Bereitstellen und Platzieren des Heizorgans in das Leiterkartenmaterial eingebracht werden. Beispielsweise können Vorstanzungen über geeignete Stanzwerkzeuge oder andere schneidende und bohrende Werkzeuge eingebracht werden. Mit anderen Worten wird das Leiterkartenmaterial durch diesen Schritt vorkonfektioniert bzw. vorstrukturiert. Die Reihenfolge des Aufbringens von Leiterbahnen und das Einbringen von Vorstanzungen können unabhängig und damit nacheinander in beliebiger Reihenfolge oder gleichzeitig durchgeführt werden.

Für den Fall, dass die Heizorgane nicht integraler Bestandteil des flexiblen Leiterkartenmaterials sind, sondern im Schritt b) als separat zu bestückende Bauelemente/Heizchips bereitgestellt werden, umfasst das Platzieren des oder jedes Heizorgans z.B. mittels geeigneter SMT-Bestückungsautomaten das Aufnehmen und Abgeben jedes Heizorgans, das z.B. ein im Wesentlichen aus Silizium bestehender Heizerchip aus einem gesägten Waferverbund oder auch ein Folienheizelement sein kann, über der oder jeder in dem Leiterkartenmaterial ausgebildeten Vorstanzung als Durchlassöffnung. Zur elektrischen Anbindung der bestückten Heizorgane an die Leiterbahnen siehe weiter unten. Das Bereitstellen und Platzieren des Heizorgans an dem oder jedem Bauplatz umfasst im Sinne der Erfindung z.B. auch das Bereitstellen und Platzieren an jedem zweiten Bauplatz, sofern dies gewünscht oder erforderlich ist, oder nur an fehlerfreien Bauplätzen. An jedem Bauplatz bedeutet daher nichts anderes, als an jedem gewünschten, vorgegebenen Bauplatz.

In dem Schritt c) wird mindestens das oder jedes Heizorgan z.B. mit geeigneten Silikonen, Kunststoffen, Polyimid, Gummi oder anderen als Dichtmaterial geeigneten Materialien teilweise umschlossen, beispielsweise mittels des so genannten Film-Assisted-Moulding-Verfahrens. Es ist auch denkbar, dass das Dichtmaterial einen Mehrschichtaufbau aus einer beliebigen Kombination der vorgenannten Materialien aufweist und durch eine mehrfache Anwendung des Film-Assisted-Moulding-Verfahrens mit jeweils einem anderen oder den gleichen aus der Liste der vorgenannten Materialien hergestellt wird. Bereiche des Heizorgans, nämlich insbesondere die Heizfläche, werden dabei partiell ausgespart. Mit dem Bilden der Ummantelung wird zum einen eine (zusätzliche) mechanische Halterung jedes Heizorgans gebildet. Zum anderen und besonders vorteilhaft werden mit der Ummantelung gleichzeitig Dichtflächen geschaffen. Besonders vorteilhaft lassen sich durch das mindestens teilweise Abdecken durch mindestens partielles Umspritzen bzw. Umformen des Bauplatzes mit Dichtmaterial zuverlässige und an gewünschte oder benötigte Konturen angepasste Dichtflächen erzielen. Die in der Verdampfereinheit integrierten Dichtflächen/Dichtungen, vorzugsweise mit einer definierten Dichtfläche, können die Anzahl benötigter Komponenten, wie z.B. zusätzliche Dichtmittel, bei der Montage zu einem Inhalator reduzieren. Dadurch vereinfacht sich die Montage und die Automatisierung des Prozesses ist optimiert. Ein weiterer Vorteil des mindestens teilweisen Abdeckens jedes Bauplatzes mit dem Dichtmaterial besteht darin, dass durch die Formgebung der Ummantelung umgebende Geometrien der Verdampfereinheit z.B. für Luft- und/oder Flüssigkeitsführung in der Verdampfereinheit inkludiert sind, nämlich integraler Bestandteil jeder Verdampfereinheit sind. Dadurch, dass das Dichtmaterial im Schritt c) aufgebracht wird, kann auch eine scharfe Abgrenzung zur Heizfläche des Heizorgans erzielt werden, so dass eine wirkungsvolle Dichtung direkt bis an die aktive Heizfläche, also den aktiven Durchlassbereich für Liquid aus einem Tank, erzielt wird. Im Schritt c) wird optional nur die die Heizorgane tragende Oberseite mit dem Dichtmaterial versehen. Die Unterseite jeder Verdampfereinheit kann frei von Dichtmaterial durch das Leiterkartenmaterial begrenzt sein.

Vorzugsweise wird im Schritt a) flexibles Leiterkartenmaterial bereitgestellt, bei dem im Bereich jedes Bauplatzes für eine einzelne Verdampfereinheit mindestens ein Bauplatz für einen RFID-Chip ausgebildet ist, wobei das flexible Leiterkartenmaterial hinsichtlich Position und/oder Verlauf vorbestimmter Leiterbahnen als RFID-Antenne vorstrukturiert ist oder vorstrukturiert wird. Damit wird ein hoher Grad an Integration erreicht, indem - ohnehin - vorhandener Bauraum/Platz auf dem Leiterkartenmaterial genutzt wird, um die Funktionalität jeder daraus gewonnenen Verdampfereinheit zu verbessern, ohne den Bauraum zu verändern und ohne die Effizienz bei der Massenherstellung negativ zu beeinflussen. Die vorbestimmten Leiterbahnen als RFID-Antenne bilden letztlich mindestens eine Spule, wobei Sende- und Empfangsspulen separat und getrennt voneinander ausgebildet sein können, vorzugsweise planparallel zueinander mit geringem Abstand, oder integrativ als eine gemeinsame Sende- und Empfangsspule ausgebildet sein können. Die bevorzugte Weiterbildung ermöglicht insbesondere auch ein modulares Produktkonzept, bei dem wahlweise ein Heizorgan und/oder ein RFID-Chip integrierbar sind.

In einer vorteilhaften Weiterbildung ist oder wird das flexible Leiterkartenmaterial mindestens mit hinsichtlich Position und/oder Verlauf vorbestimmten Leiterbahnen und/oder Vorstanzungen für jeden Bauplatz zur elektronischen Anbindung mindestens des RFID-Chips wahlweise vorstrukturiert. Dadurch werden bereits beim Strukturierungsprozess des Leiterkartenmaterials die Anschlüsse und/oder die Antenne selbst vorgesehen und eingearbeitet, was eine besonders kompakte und effiziente Herstellung der Verdampfereinheiten gewährleistet.

Vorzugsweise wird in einem Schritt k), der vor, mit oder nach dem Schritt b) erfolgen kann, an jedem Bauplatz ein RFID-Chip bereitgestellt und platziert. Das erfolgt für den Fall, dass der oder jeder RFID-Chip nicht integraler Bestandteil des flexiblen Leiterkartenmaterials ist, sondern im Schritt k) als separat zu bestückendes Bauelement bereitgestellt wird. Als RFID-Chip können z.B. NFC-Komponenten bereitgestellt und platziert werden. Andere Kurzdistanz-Funkmodule sind aber ebenfalls einsetzbar. Der oder jeder RFID-Chip kann selbst über eine Antenne verfügen, um mit der oder jeder RFID-Antenne kommunizieren zu können. Das Platzieren des oder jedes RFID-Chips erfolgt z.B. mittels geeigneter SMT-Bestückungsautomaten, ebenso wie das Aufnehmen und Abgeben jedes RFID-Chips. Zur elektrischen Anbindung der bestückten RFID-Chips an Leiterbahnen siehe weiter unten.

Wie erwähnt, kann der RFID-Chip integraler Bestandteil des Leiterkartenmaterials sein. Optional wird der separate RFID-Chip bereitgestellt, platziert und elektrisch mit den Leiterbahnen verbunden, wobei der RFID-Chip an jedem Bauplatz auch mit den die RFI D-Antenne bildenden Leiterbahnen wahlweise verbunden oder verbindbar ist. Die Verbindung kann elektrisch erfolgen oder über Funksignale.

Um den RFID-Chip zu schützen, insbesondere vor Feuchtigkeit, wird optional im Schritt c) zusätzlich zum teilweise Abdecken jedes Bauplatzes für das Heizorgan auch der Bauplatz für den RFID-Chip mit dem Dichtmaterial unter Bildung von Dichtflächen abgedeckt.

Vorzugsweise erfolgt das Bereitstellen des flexiblen Leiterkartenmaterials in Schritt a) wahlweise durch Abrollen des auf Rollen gelagerten Leiterkartenmaterials oder durch Zuführen von in Magazinen oder dergleichen bevorrateten Abschnitten des Leiterkartenmaterials. Die Rollen mit flexiblem Leiterkartenmaterial sind platzsparend zu bevorraten und ermöglichen ein schnelles Bereitstellen der Bauplätze in großer Anzahl durch einfaches Abrollen/Abwickeln. Entsprechendes gilt für das Zuführen von Abschnitten des Leiterkartenmaterials aus Magazinen oder dergleichen.

Vorteilhafterweise wird im Schritt c) jeder Bauplatz mindestens teilweise derart mit Dichtmaterial umschlossen, dass zusätzlich sowohl auf der Oberseite als auch auf einer der Oberseite gegenüberliegenden Unterseite des flexiblen Leiterkartenmaterials an den Außenflächen der Ummantelung Dichtflächen für eine frei von zusätzlichen Dichtmitteln bestehende Verbindung zu den die Verdampfereinheit umgebenden Komponenten des Inhalators ausgebildet werden. Das Bilden zusätzlicher, vorzugsweise definierter, Dichtflächen insbesondere auf der Unterseite des Leiterkartenmaterials kann auch vor/während/nach dem Auftragen des Dichtmaterials auf die Oberseite erfolgen. Damit ist die Verdampfereinheit besonders einfach und besonders dichtend montierbar, z.B. in standardisierte Verdampferbaugruppen oder dergleichen.

Wie weiter oben beschrieben, können die Heizorgane als separate Bauelemente bereitgestellt werden. Für diesen Fall ist es besonders vorteilhaft, dass der Schritt b) als Schritt b₁) das Auftragen von elektrischem Kontaktierungsmaterial mindestens auf Kontaktflächen der Leiterbahnen an jedem Bauplatz auf der Oberseite des flexiblen Leiterkartenmaterials, als Schritt b₂) das Platzieren des mindestens einen Heizorgans im Bereich jedes Bauplatzes, derart, dass eine die Oberseite mit der Unterseite des Leiterkartenmaterials verbindende Vorstanzung vollständig oder teilweise durch das oder jedes Heizorgan abgedeckt ist, und als Schritt b₃) das Ausbilden einer elektrischen Verbindung zwischen Heizorgan und Leiterbahnen an jedem Bauplatz umfasst.

Wie weiter oben beschrieben, können die RFID-Chips als separate Bauelemente bereitgestellt werden. Optional umfasst der Schritt k) als Schritt k₁) das Auftragen von elektrischem Kontaktierungsmaterial mindestens auf Kontaktflächen der Leiterbahnen an jedem Bauplatz für die RFID-Chips auf der Oberseite des flexiblen Leiterkartenmaterials, als Schritt k₂) das Platzieren des mindestens einen RFID-Chips im Bereich jedes Bauplatzes, und als Schritt k₃) das Ausbilden einer elektrischen Verbindung zwischen RFID-Chip und Leiterbahnen an jedem Bauplatz. In weiteren Ausführungsformen bzw. bevorzugten Weiterbildungen kann elektrisches Kontaktierungsmaterial auch auf die Kontakte des RFID-Chips aufgebracht werden. Konkret kann beispielsweise anisotrop leitfähiger Klebstoff auf die Unterseite des Chips im Bereich der Kontakte aufgebracht werden, wobei die derart vorbereiteten Chips auf die Leiterbahnen des flexiblen Leiterkartenmaterials platziert werden.

Das Auftragen des elektrischen Kontaktierungsmaterials, das z.B. Lötpaste, Lot, Sinterpaste, elektrisch leitender Klebstoff oder dergleichen oder eine Kombination eines oder mehrerer der vorgenannte Kontaktierungsmaterialien sein kann, kann mittels geeigneter Verfahren erfolgen. Beispiele für geeignete Verfahren sind Siebdruck, Schablonendruck, Tampondruck, Dispensen oder dergleichen oder eine Kombination eines oder mehrerer der vorgenannten Verfahren. Diese Verfahren werden mit Hilfe von geeigneten Vorrichtungen durchgeführt. Ein Beispiel für eine geeignete Vorrichtung ist ein Siebdruckautomat.

Das Platzieren des Heizorgans und auch des RFID-Chips kann vorzugsweise mit einem SMT-Bestückungsautomaten oder dergleichen erfolgen. Das Ausbilden der elektrischen Verbindung kann z.B. durch Umwandeln des Kontaktierungsmaterials z.B. durch Wärme und/oder Druck in einem Ofen, einer Sinterpresse, Kontakt-Thermoden oder dergleichen oder einer Kombination einer oder mehrerer der vorgenannten Varianten erfolgen. Optional ist im Schritt b₃) auch das Verdrahten mittels des so genannten Wirebondings möglich. Durch die Adaptierung des Herstellungsprozesses der Verdampfereinheiten an standardisierte Fertigungsprozesse und Fertigungsvorrichtungen insbesondere in Schritt b) ist zum einen eine Massenproduktion von Verdampfereinheiten gewährleistet, und zum anderen können Kapazitätsengpässe, die beim Einsatz von Sonderverfahren/Sondermaschinen auftreten können, zuverlässig vermieden werden.

Es ist auch denkbar, dass das so genannte NanoWiring-Verfahren zur elektrischen Kontaktierung des Heizorgans verwendet wird. Beim NanoWiring-Verfahren werden Nanodrähte auf einer Oberfläche aufgewachsen. Beim NanoWiring-Verfahren handelt es sich um einen galvanischen Prozess, der dem Tampon-Druck ähnelt. Hierbei wird ein Schwamm, der einen Elektrolyten trägt, auf das Substrat gepresst. Ein metallischer Rasen mit Durchmessern von wenigen Nanometern bis einigen Mikrometern und Längen von einige Hundert Nanometern bis einigen zehn Mikrometern wächst dabei in die Porösitätsschicht des Schwammes hinein. Durch einen Strukturierungsprozess (auch Maskieren genannt) werden die Substrate nur in den vorgesehenen Bereichen mit NanoWiring-Beschichtung beschichtet. Zum Schluss werden beim Stripping-Prozess alle Materialien entfernt, die nicht zur Verbindung benötigt werden und eine Reinigung des Substrates vorgenommen. Durch den galvanischen Prozess ist es möglich, die Nanodrähte aus praktisch allen galvanisch abscheidbaren Metallen herzustellen. Beispielsweise kann Kupfer, Gold oder Nickel für die Herstellung der NanoWiring-Beschichtung verwendet werden.

Beim sogenannten KlettWelding werden zwei mittels des NanoWiring-Verfahrens vorbereitete Substrate, d.h. das Abschnitte der Leiterbahnen auf dem Leiterkartenmaterial und Abschnitte auf dem Heizorgan, die jeweils mit einem NanoWiring-Beschichtung und damit mit Nanodrähten versehen sind, durch Zusammendrücken (z.B. mit 20 MPa) bei Raumtemperatur verbunden. Diese Kraft kann in Abhängigkeit der Bauteilgröße von handelsüblichen Bestückern bzw. FlipChip-Bondern aufgebracht werden. Großflächige Verbindungen werden mit elektromotorischen oder hydraulischen Kraftmaschinen hergestellt. Ebenfalls ist die Anwendung von einfachen Kniehebelpressen möglich. Durch ihren kleinen Durchmesser verbinden sich die einzelnen Drähte instantan mechanisch und zusätzlich auf Atomgitterebene - ähnlich wie beim Kaltverschweißen. Die resultierende Verbindung hat vergleichbare elektrische - und thermische - Kenndaten wie gewalztes Kupfer, bei gleichzeitig hoher mechanischer Festigkeit.

Es ist auch denkbar, dass die elektrische Verbindung mittels des sogenannten KlettSintering-Prozesses erfolgt. Dabei weist nur ein Substrat, d.h. entweder das Leiterkartenmaterial oder das Heizorgan, eine NanoWiring-Beschichtung auf. Das zweite Substrat, d.h. entweder das Heizorgan oder das Leiterkartenmaterial, benötigt eine verkupferte oder vergoldete Oberfläche. Beide Substrate werden zusammengedrückt (z.B. mit 20 MPa) und eine Temperatur im Bereich von 210 °C wird in die Verbindungszone z.B. über eine Thermode gebracht. Entsprechendes gilt auch für die elektrische Kontaktierung des RFID-Chips.

In einer bevorzugten Weiterbildung des Verfahrens werden nach dem Schritt c) die beim Abdecken bzw. Umschließen der Bauplätze mit Dichtmaterial entstehenden Angussabschnitte des Dichtmaterials in einem Schritt d) entfernt. Damit wird jeder Bauplatz bzw. die darauf gebildete Verdampfereinheit - mit Ausnahme mindestens eines Verbindungsstegs - vom Leiterkartenmaterial und sämtlichen weiteren Verbindungen, die nicht Bestandteil einer Verdampfereinheit sind, gelöst bzw. getrennt. Es ist aber auch denkbar, dass das Lösen bzw. Trennen der Angussabschnitte gleichzeitig bei einer Vereinzelung der Verdampfereinheiten aus dem Gurt aus dem Leiterkartenmaterial erfolgt.

Vorzugsweise kann nach dem Schritt c) oder d) das mit der Vielzahl fertiggestellter, dichtender Verdampfereinheiten versehene Leiterkartenmaterial zur weiteren Verwendung in einem Schritt e) wahlweise wieder auf eine Rolle aufgerollt oder in einem Magazin oder dergleichen gesammelt werden. Das Aufrollen/Sammeln kann auch schon nach dem Schritt b) erfolgen, insbesondere ab dem Zeitpunkt der dauerhaften Verbindung zwischen Heizorgan und Leiterkartenmaterial. Die weitere Verwendung kann ein Bevorraten, Zwischenspeichern oder eine weitere Verarbeitung sein. Durch das zwischenzeitliche Aufrollen/Sammeln des in den Schritten a) und b) bzw. a) bis c) bzw. d) hergestellten Leiterkartenmaterials mit darauf erzeugten Verdampfereinheiten wird eine platzsparende, effektive Massenherstellung von Verdampfereinheiten wirksam unterstützt. Beispielsweise lassen sich komplette Rollen oder Magazine oder dergleichen mit einer Vielzahl von Verdampfereinheiten ausliefern, so dass im Folgenden eine massenweise Montage der Verdampfereinheiten nach erfolgter Vereinzelung an einem beliebigen Ort ermöglicht wird.

Zur Vorbereitung der Montage der Verdampfereinheiten werden die dichtenden Verdampfereinheiten zur weiteren Verwendung in einem Schritt f) von dem flexiblen Leiterkartenmaterial vereinzelt. Das Vereinzeln kann z.B. bevorzugt durch Stanzen von dem Leiterkartenmaterial erfolgen, indem der oder jeder verbliebene Verbindungssteg zum Leiterkartenmaterial getrennt wird. Die Verdampfereinheiten können auch aus dem Vollen aus dem Leiterkartenmaterial getrennt werden, also ohne ganz oder teilweise umlaufende Vorstanzungen. Auch Sägen oder Schneiden sind Optionen zum Vereinzeln. Die vereinzelten Verdampfereinheiten können dann als Tray- oder Bulkware weiterverarbeitet oder direkt weiter montiert werden

Zur Vervollständigung der Verdampfereinheit kann in einem Schritt g) auf oder an der Oberseite des flexiblen Leiterkartenmaterials auf den freien Heizflächen der Heizorgane an jedem Bauplatz mindestens ein Dochtorgan platziert werden. Bevorzugt wird das Dochtorgan auf oder an einer Oberseite (O) des Leiterkartenmaterials montiert. Das Dochtorgan kann insbesondere bei einer einstückigen Ausbildung z.B. mittels SMT-Bestückungsautomat auf der freien Heizfläche platziert werden. Das Dochtorgan kann auch als granulares Dochtorgan ausgestaltet sein und zur Montage geschüttet werden. Ein granulares Dochtorgan ist wenigstens zum Teil aus einer Vielzahl granulatartiger Körner gebildet, die aufgrund ihrer Schüttung und/oder Ausbildung Mikrokanäle bilden. Für den Fall, dass das Dochtorgan bereits im Verbund mit dem Heizorgan ausgebildet ist, das Heizorgan also gleichzeitig das Dochtorgan darstellt oder das Heizorgan an und/oder auf der Heizfläche ein Dochtorgan aufweist, wird durch das Platzieren des Heizorgans gleichzeitig auch das Dochtorgan platziert. Das Dochtorgan kann entsprechend zusammen mit dem Schritt b) oder nach dem Schritt b) in beliebiger Schrittfolge platziert werden. Beim Platzieren nach dem Schritt b) kann das oder jedes Dochtorgan noch vor dem Schritt c) oder nach dem Schritt c) platziert werden. Zum Fixieren jedes Dochtorgans kann dieses z.B. nach dem Schritt c) in eine durch das Dichtmaterial gebildete Ausnehmung/Tasche oder dergleichen platziert/gedrückt/montiert/fixiert werden. Das Dochtorgan kann auch nach dem Schritt c) auf der freien Heizfläche platziert und durch Einschieben in eine Aufnahme, z.B. ausgebildet in einer Komponente einer Verdampferbaugruppe, fixiert werden. Letztlich kann der Zeitpunkt des Bestückens/Platzierens der Dochtorgane nahezu beliebig ausgewählt werden. Selbst das Platzieren des Dochtorgans nach dem Vereinzeln der Verdampfereinheiten vom Leiterkartenmaterial ist möglich.

In einer bevorzugten Weiterbildung des Verfahrens kann in einem Schritt h), vorzugsweise zusammen mit dem Schritt b₂) und k₂) mindestens ein zusätzliches elektronisches Bauteil im Bereich jedes Bauplatzes platziert werden. Das elektronische Bauteil ist beispielsweise ein ID-Chip, der als Kennelement zur eindeutigen Identifizierung der jeweiligen Verdampfereinheit dient und als NFC-Element, RFID-Element oder als digitaler Speicherbaustein (z.B. EEPROM) ausgebildet sein kann. Der oder jeder ID-Chip kann optional auch mit einer Antenne, beispielsweise einer NFC-Antenne oder einer RFID-Antenne, verbunden sein. Hinsichtlich der Position des oder jedes ID-Chips auf dem Bauplatz sind unterschiedliche Optionen möglich. Es besteht auch die Möglichkeit, die zusätzlichen Komponenten, es können anstelle des ID-Chips oder ergänzend zum ID-Chip, auch Sensoren und andere elektronischen Bauteile platziert werden, nicht nur auf der Oberseite, sondern auch auf der Unterseite im Bereich entsprechender elektrischer Kontaktflächen zu positionieren. Der ID-Chip und jede andere Komponente können auch durch die Ummantelung mindestens teilweise mit abgedeckt oder umschlossen werden. Das Abdecken/Umschließen kann mit dem Dichtmaterial erfolgen, mit dem auch das Heizorgan abgedeckt/umschlossen wird, insbesondere dann, wenn das elektronische Bauteil vor dem Schritt c) platziert wird. Das Abdecken/Umschließen kann aber auch in einem separaten Schritt mit demselben Dichtmaterial oder einem zweiten Material, insbesondere einem Dichtmaterial, erfolgen.

Vorzugsweise wird vor dem Schritt h), und besonders vorzugsweise zusammen, also gleichzeitig mit Schritt b₁) und k₁) Kontaktierungsmaterial auf Kontaktflächen der Leiterbahnen an jedem Bauplatz auf der Oberseite und/oder der Unterseite des flexiblen Leiterkartenmaterials aufgebracht, und nach dem Schritt h), vorzugsweise zusammen, also gleichzeitig mit Schritt b₃) bzw. k₃) eine elektrische Verbindung zwischen dem oder jedem elektronischen Bauteil und Leiterbahnen an jedem Bauplatz ausgebildet. Entsprechend können die für die Heizorgane eingesetzten Fertigungsautomaten eingesetzt werden.

Besonders bevorzugt wird jedes Heizorgan und/oder jeder RFID-Chip und/oder jedes zusätzliche elektronische Bauteil elektrisch mit den Leiterbahnen des flexiblen Leiterkartenmaterials verbunden durch Einsatz eines oder mehrerer Verfahren aus der Liste: Direktkontaktierung mittels Silber-Sintern, eutektisch Bonden, leitfähig Kleben, anisotrop leitfähig Kleben, KlettWelding, KlettSintering, Löten, Schweißen. Dadurch wird - mit ausreichender thermischer Isolierung - eine besonders gute Kontaktierung zu den Leiterbahnen erreicht.

Optional werden im Schritt c) beim Abdecken bzw. Umschließen jedes Bauplatzes aus und/oder mit dem Dichtmaterial auf der Unterseite des Leiterkartenmaterials an jedem Bauplatz mindestens Abschnitte eines Luftströmungskanals gebildet. Das Bilden der Abschnitte des Luftströmungskanals kann auch vor dem Schritt c) und nach dem Schritt c) erfolgen, und zwar durch das Dichtmaterial, mit dem auch das Heizorgan abgedeckt/umschlossen wird, oder mit einem zweiten Dichtmaterial. Diese Ausführungsform führt zu einer so genannten "offenen" Variante, da der Luftströmungskanal erst in Kombination mit die Verdampfereinheit umgebenden Komponenten zu einem durchgängigen, umfangsseitig geschlossenen Luftströmungskanal wird.

In einer weiteren Option kann an jedem Bauplatz ein rohrförmiger, vorkonfektionierter Abschnitt als Teil eines Luftströmungskanals auf der Unterseite des Leiterkartenmaterials platziert und mittels zusätzlichem Dichtmaterial umschlossen und damit fixiert werden. Diese Ausführungsform führt zu einer so genannten "geschlossenen" Variante, da die Verdampfereinheit selbst einen durchgängigen, umfangsseitig geschlossenen Abschnitt des Luftströmungskanals umfasst.

Besonders bevorzugt werden mindestens die Schritte a) bis c) nacheinander jeweils an einer eigenen oder der gleichen Fertigungsstation einer Fertigungslinie ausgeführt, derart, dass bei jedem Schritt eine Vielzahl von Bauplätzen zeitgleich bearbeitet wird. Dadurch ist ein hoher Automatisierungsgrad für eine schnelle und effiziente Großserienfertigung mit einer stark optimierten Teilelogistik gewährleistet.

Besonders bevorzugt erfolgt der gesamte Herstellungsprozess der Verdampfereinheiten in einer gemeinsamen Fertigungslinie mit mehreren Fertigungsstationen, wodurch die vorstehenden Vorteile noch weiter unterstützt werden.

In einer bevorzugten Weiterbildung wird das flexible und vorzugsweise aus Polyimid bestehende, vorstrukturierte Leiterkartenmaterial als Endlosstrang von einer Vorratsrolle abgewickelt und kontinuierlich oder intermittierend durch die mehrere Fertigungsstationen umfassende Fertigungslinie transportiert. Damit besteht die Möglichkeit, beispielsweise den Endlosstrang abzurollen, so dass z.B. an einer ersten Fertigungsstation der Schritt b) an einer Vielzahl von Bauplätzen für eine Vielzahl von Verdampfereinheiten ausgeführt wird. Nach dem Fertigstellen des Schritts b) kann der Endlosstrang weitertransportiert werden, um einen nachfolgenden Abschnitt des Endlosstrangs zur Ausführung des Schritts b) in den Bereich der ersten Fertigungsstation zu transportieren. Optional kann sich dann z.B. der vorauslaufende Abschnitt, der bereits dem Verfahrensschritt b) unterzogen wurde, in einer zweiten Fertigungsstation zum Ausführen des Schritts c) befinden. Diese Prozessfolge kann durchgängig bis zum Schritt h) und einschließlich Schritt k) ausgeführt werden. Somit können alle Herstellungsschritte mit Hilfe einer Fertigungslinie vorzugsweise vollständig automatisiert ausgeführt werden. Das Verfahren kann entsprechend auch durchgeführt werden, indem Abschnitte des flexiblen Leiterkartenmaterials mit einem Bauplatz oder mehreren Bauplätzen z.B. in Magazinen, Kassetten oder dergleichen zugeführt und mit oder ohne Magazin oder dergleichen durch die Fertigungslinie transportiert werden.

Die Aufgabe wird gemäß einem zweiten Aspekt der Erfindung insbesondere auch durch eine eingangs genannte Verdampfereinheit gelöst, die gekennzeichnet ist durch eine aus Dichtmaterial bestehende Ummantelung, die den Leiterkartenabschnitt und das Heizorgan unter Freihaltung einer Heizfläche mindestens teilweise abdeckt und nach außen hin mit Dichtflächen versehen ist. Das flexible Leiterkartenmaterial des Leiterkartenabschnitts bietet neben der Möglichkeit der Massenfertigung eine besonders energieeffiziente Ausbildung des Querschnitts der Leiterbahnen. Durch die aus Dichtmaterial bestehende Ummantelung des Heizorgans ist dieses zum einen sicher mechanisch gehalten und zum anderen führen die nach außen hin, also vom Heizorgan wegweisenden, vorzugsweise definierten Dichtflächen dazu, dass die Verdampfereinheit insgesamt eine Doppelfunktion beinhaltet, nämlich einerseits als Verdampfer und andererseits als Dichtmittel, so dass mit der erfindungsgemäßen Verdampfereinheit mit integriertem Dichtmittel eine teilereduzierte Montage gewährleistet ist. Insgesamt ist durch die erfindungsgemäße Ausbildung eine energieeffiziente, zuverlässige und für die Massenproduktion taugliche Verdampfereinheit geschaffen.

Vorteilhafterweise umfasst der Leiterkartenabschnitt mindestens einen RFID-Chip und mindestens eine RFID-Antenne. Damit sind der oder jeder RFID-Chip und die oder jede RFID-Antenne integrativer Bestandteil des Trägersubstrats und damit der Verdampfereinheit selbst, so dass auf kompakte und effiziente Weise eine kontaktlose Übertragung von Daten z.B. zwischen der Verdampfereinheit bzw. einer die Verdampfereinheit umfassenden Verdampferkartusche und einem Kartuschenträger oder zwischen der Verdampfereinheit bzw. einem die Verdampfereinheit umfassenden Inhalator und einem Auslesegerät, wie z.B. einem Smartphone, sichergestellt ist. Typische Daten, die übertragen werden können, sind z.B. Daten zur korrekten Verdampfung, zur Berechtigung des Nutzers des Inhalators, zur Aktivierung der Verdampferkartusche oder zum Verfolgen des Nutzungsverhaltens durch den Nutzer selbst und/oder einen Arzt oder Apotheker insbesondere für medizinisch genutzte Inhalatoren.

Vorteilhafterweise ist der Leiterkartenabschnitt aus Polyimid gebildet, ein dotierter Siliziumchip mit Mikrokanälen ist als Heizorgan durch Direktkontaktierung mittels Silbersintern elektrisch mit den Leiterbahnen verbunden, und der Leiterkartenabschnitt sowie der Siliziumchip sind teilweise von Silikon oder Polyimid umschlossen, so dass einerseits der Siliziumchip mechanisch gehalten ist und andererseits nach außen hin vorzugsweise definierte Dichtflächen gebildet sind. Die Dichtflächen können undefiniert ausgebildet sein. Bevorzugt weisen die Dichtflächen aber vorgegebene und reproduzierbare, also definierte Dichtflächen auf. Eine Alternative zu Silikon kann ein geeigneter Kunststoff oder Polyimid sein. Zudem sind Leiterkartenabschnitt und Siliziumchip derart von dem Dichtmaterial umschlossen, dass Flüssigkeit durch die Mikrokanäle des Siliziumchips von einer ersten Seite des Siliziumchips zu einer zweiten Seite des Siliziumchips transportierbar ist. Mit dieser Ausbildung werden die zuvor beschriebenen Vorteile noch weiter verstärkt. Alternativ zum Siliziumchip kann auch ein so genannter Folienheizer als Heizorgan eingesetzt werden bzw. vorgesehen sein, der eine Mikrostruktur aufweist, mittels der Flüssigkeit von einer ersten Seite des Folienheizers zu einer zweiten Seite des Folienheizers transportierbar ist.

In einer besonders bevorzugten Weiterbildung stehen die in dem flexiblen Leiterkartenmaterial eingebetteten Leiterbahnen aus der durch das Dichtmaterial gebildeten Ummantelung zur Bildung einer Flexkontaktierung biegbar, insbesondere reversibel und/oder flexibel biegbar, hervor. Während und nach dem Biegen sind die in das Leiterkartenmaterial eingebetteten Leiterbahnen elektrisch leitend und kontaktieren den Siliziumchip oder den Folienheizer oder jedes andere Heizorgan. Dadurch kann auf weitere Kontaktierungen insbesondere in die Verdampfereinheit umgebenden Komponenten, beispielsweise einer Verdampferbaugruppe oder Verdampferkartusche, verzichtet werden, was die Teilevielfalt weiter reduziert und insbesondere eine stark vereinfachte und platzsparende Montage ermöglicht.

Eine zweckmäßige Ausführungsform ist dadurch gekennzeichnet, dass die Heizfläche des Heizorgans auf der Oberseite mindestens teilweise von einem Dochtorgan abgedeckt ist. Dadurch ist die Verdampfereinheit für eine besonders effiziente und zuverlässige Nutzung vervollständigt.

Eine optionale Ausführungsform zeichnet sich dadurch aus, dass auf der Unterseite des Leiterkartenabschnitts mindestens ein Rohrabschnitt als Abschnitt eines Luftströmungskanals ausgebildet und/oder angeordnet ist, wobei der Rohrabschnitt eine zum Heizorgan gerichtete Öffnung aufweist. Der Rohrabschnitt kann aus Dichtmaterial und/oder einem separaten Rohr gebildet sein.

Besonders bevorzugt ist die Verdampfereinheit mit einem Verfahren nach einem oder mehreren der Ansprüche 1 bis 23 hergestellt. Die sich daraus ergebenden Vorteile wurden bereits im Zusammenhang mit dem Herstellungsverfahren oben erläutert, so dass zur Vermeidung von Wiederholungen auf die entsprechenden Passagen verwiesen wird.

Die Aufgabe wird gemäß einem dritten Aspekt der Erfindung insbesondere auch durch eine eingangs genannte Verdampferbaugruppe gelöst, die eine Verdampfereinheit nach dem zweiten Aspekt der Erfindung sowie einen Adapterstecker aufweist, in den die Verdampfereinheit eingesteckt ist, wobei die Verdampfereinheit bzw. deren Ummantelung zumindest teilweise mit einer Außenfläche der Ummantelung dichtend an einer inneren Geometrie des Adaptersteckers anliegt. Die innere Geometrie des Adaptersteckers bezeichnet hier Flächen eines Bereichs in dem Adapterstecker, der zur Aufnahme der Verdampferbaugruppe eingerichtet ist. Durch diese Ausbildung der Ummantelung in Verbindung mit der inneren Geometrie des Adaptersteckers besteht ohne zusätzliche Dichtmittel eine Fluidverbindung ausschließlich über die Verdampfereinheit in Richtung des Strömungskanals. Mit anderen Worten ist die Verdampferbaugruppe, mit Ausnahme der Verdampfereinheit im Bereich der Heizfläche zum Aufnehmen von Flüssigkeit aus einem Vorratstank und Erzeugen von Dampf in Richtung eines Luftströmungskanals, dicht ausgebildet.

Vorteilhafterweise umfasst die Verdampferbaugruppe mindestens einen Abschnitt eines Luftströmungskanals, der durch die Verdampfereinheit und/oder den Adapterstecker gebildet ist. Der Adapterstecker kann einen eigenen Abschnitt eines Luftströmungskanals aufweisen, wobei der Luftströmungskanal eine Öffnung für eine Verbindung zur Verdampfereinheit aufweist. Der Luftströmungskanal kann aber auch ganz durch die Verdampfereinheit oder teilweise durch die Verdampfereinheit in Kombination z.B. mit einer Wandung des Adaptersteckers bzw. einem Abschnitt der oben beschriebenen inneren Geometrie des Adaptersteckers gebildet sein.

Die Aufgabe wird gemäß einem vierten Aspekt der Erfindung insbesondere auch durch eine eingangs genannte Verdampferkartusche gelöst, die einen Hohlkörper aufweist mit mindestens einem Abschnitt eines Luftströmungskanals, einen Vorratstank zum Bevorraten von Flüssigkeit, sowie eine Verdampferbaugruppe nach dem dritten Aspekt der Erfindung, wobei die Verdampferbaugruppe mit dem Hohlkörper und dem Vorratstank derart dichtend verbunden ist, dass der Abschnitt des Luftströmungskanals der Verdampferbaugruppe und der Abschnitt des Luftströmungskanals des Hohlkörpers einen gemeinsamen Luftströmungskanal bilden und der Vorratstank mindestens eine Zugangsöffnung zum Strömungskanal aufweist, in der die Verdampfereinheit platziert ist.

Die Aufgabe wird gemäß einem fünften Aspekt der Erfindung insbesondere auch durch einen eingangs genannten Inhalator gelöst, der einen mindestens eine elektronische Steuereinheit und eine elektrische Energiequelle umfassenden Kartuschenträger sowie eine Verdampferkartusche nach dem vierten Aspekt der Erfindung aufweist.

Etliche der sich im Zusammenhang mit der Verdampferbaugruppe, der Verdampferkartusche und dem Inhalator ergebenden Vorteile wurden bereits im Zusammenhang mit dem Herstellungsverfahren sowie der Verdampfereinheit beschrieben, so dass zur Vermeidung von Wiederholungen auf die entsprechenden Passagen verwiesen wird.

Des Weiteren können die vorgenannten Vorrichtungen, vor allem der Inhalator, insbesondere im Hinblick auf medizinische Anwendungen so eingerichtet sein oder eingesetzt werden, dass eine oder mehrere der folgenden weiteren Vorteile auftreten:
Während bei bekannten Inhalatoren für pulverförmige Medikamente der Inhalator in der Regel vor und/oder nach der Benutzung gereinigt oder gar darüberhinausgehend gewartet werden muss, ist dies bei einem erfindungsgemäßen Inhalator in der Regel nicht erforderlich, insbesondere da das Medikament, in flüssiger Form in der Verdampferkartusche bzw. deren Vorratstank eingeschlossen ist.

Zudem kann ein erfindungsgemäßer Inhalator für verschiedene Therapien zum Einsatz kommen, wozu entweder die Verdampferkartusche mit einer Flüssigkeit mit zumindest einem entsprechend therapiebezogenen Wirkstoff im Hinblick auf die anzuwendende Therapie entsprechend neu eingebracht oder eine bereits montierte Verdampferkartusche gegen eine andere zur vorzunehmenden Therapie passenden Verdampferkartusche mit entsprechendem Inhalt ausgetauscht wird.

Auch kann ein individuelles, insbesondere manuelles Beladen und vorausgehendes oder nachfolgendes Reinigen des Inhalators je Dosis oder Anwendung entfallen, wenn der Vorratstank der Verdampferkartusche so groß gestaltet ist, dass er über genug Raum für die Aufnahme von einer für mehrere Dosen bzw. Anwendungen ausreichenden Menge der zu verdampfenden Flüssigkeit verfügt.

Da bei dem Inhalator das Medikament in Form von Dampf in der Regel vollständig in den zur Inhalation dienenden (Atem-) Luftstrom des Benutzers abgegeben wird, kann eine Reihe von weiteren Vorteilen realisiert werden: Insbesondere treten typischerweise keine im Inhalator verbleibenden Reste des Medikaments nach der Anwendung auf, weil der Medikamentendampf, zumindest im Wesentlichen, nur im Luftstrom rekondensiert. Außerdem lässt sich ein besonders guter Anwendungserfolg erzielen, da die maximal durch den Patienten aufnehmbare Medikamentenmenge in den inhalierbaren Luftstrom abgebbar ist. Auch kann so eine Überdosierung des Medikaments durch Aufnahme von nicht entferntem Medikament bei nachfolgender Anwendung des Inhalators vermieden werden, was insbesondere im Hinblick auf eine gesteigerte Sicherheit für den Patienten vorteilhaft ist. Auch lässt sich so eine Steigerung der Kontrollmöglichkeiten bei der Behandlung des Patienten erreichen, da eine genauere Dosierung möglich ist.

Zudem kommt der erfindungsgemäße Inhalator ohne Dispergiermittel und Treibmittel (insbesondere Treibgase) aus, die insbesondere im medizinischen Umfeld oftmals ungünstig oder gar gesundheitsgefährdend sein können. Unter einem Treibgas ist hierbei insbesondere ein Gas zu verstehen, das einen gegenüber dem Umgebungsdruck erhöhten Druck aufweist und das in typischen herkömmlichen Inhalatoren dazu dient, das zu applizierende Medikament zu zerstäuben und zu beschleunigen. Auch kann auf eine Kompressionseinrichtung zur Erzeugung eines Luft- oder anderen Gasstroms, ebenso wie auf damit einhergehende Totvolumen im Gehäuse des Inhalators verzichtet werden. So lässt sich eine Verringerung des erforderlichen Bauraums erreichen, da nur ein Luftkanal benötigt wird anstatt raumfordernder Druckkammern oder aufwendiger Federsysteme zum Vorspannen des Inhalators. Die geringeren Bauraumanforderungen können wiederum insbesondere im Hinblick auf eine so ermöglichte Steigerung der Gestaltungsfreiheiten beim Design des Inhalators (z.B. kleinere, ansprechendere Designs sind möglich) vorteilhaft sein.

Bei einem erfindungsgemäßen Inhalator kann der Benutzer dagegen das Medikament durch eigene Atemluft in die Lunge überführen, nachdem das Medikament verdampft wurde. Aufgrund des ermöglichten Verzichts auf einen Einsatz von Treibmitteln zum Zerstäuben des Medikaments können bei der Benutzung herkömmlicher Inhalatoren mit Treibmitteln häufig auftretende Probleme beim Patienten, wie z.B. eine eine Reizung des Rachens oder Husten, effektiv vermieden werden. Auch entfällt ein bei herkömmlichen Inhalatoren sonst oftmals erforderliches manuelles Vorspannen des Inhalators zum Zerstäuben des Medikaments. So lässt sich die Benutzerfreundlichkeit insbesondere durch Vermeiden von manuellen Vorbereitungshandlungen vor der Anwendung des Inhalator verbessern.

Die erfindungsgemäß vorgesehene Dichtung hat zudem den Vorteil, dass einem unerwünschten oder wirkstoffabhängig möglicherweise sogar potentiell schädlichen Eintreten von unverdampfter Flüssigkeit in den Luftkanal mit der damit verbundenen Möglichkeit der Aufnahme der Flüssigkeit als solche durch den Benutzer effektiv begegnet wird und somit die Anwendungssicherheit des Inhalators sichergestellt wird.

Da der erfindungsgemäße Inhalator, insbesondere dessen Heizorgan, elektrisch betrieben wird, lässt sich eine hohe Dosiergenauigkeit des Heizorgans aufgrund der genauen Steuerbarkeit des Heizbetriebs erreichen. Dies fördert insbesondere die Anwendungsqualität, indem die verdampfte Menge des Medikaments an die individuellen Bedürfnisse des Benutzers bzw. Patienten sehr gut anpassbar und insbesondere auch im Sinne einer maximalen Dosis begrenzbar ist, was wiederum zu einer Steigerung der Anwendungssicherheit bei der Benutzung des Inhalators genutzt werden kann.

Bevorzugt kann ein erfindungsgemäßer Inhalator eine Ausgabemengenkontrolleinrichtung aufweisen. Dabei kann es sich insbesondere um eine Zähleinrichtung handeln, insbesondere um eine Zähleinrichtung zum Zählen von in einem definierten Betrachtungszeitraum (beispielsweise im Zeitraum seit einem letzten Initialisieren bzw. Zurücksetzen der Zähleinrichtung) verdampften Dosen der Flüssigkeit aufweisen. Als zu zählende Dosen kann hier insbesondere (i) eine Anzahl von verdampften Füllungen der Verdampferkartusche bzw. von deren Vorratstank bzw. von verschiedenen Verdampferkartusche bzw. von deren jeweiligen Vorratstanks, oder (ii) eine Anzahl von durch den Inhalator im betrachteten Zeitraum ausgegebenen Dampfstößen oder vorbestimmten Dampfmengeneinheiten in Frage kommen. So lässt sich, insbesondere bei medizinischen Anwendungen, im Hinblick auf die Einhaltung einer gewünschten Dosierung die Ausgabemenge des verdampften Stoffes erfassen und somit eine einfache und zuverlässige Überwachung der Dosierung realisieren. Zudem ist aufgrund dieser Kontrollmöglichkeit im Hinblick auf die Verwendung einzelner Dosen ein individuelles Beladen des Inhalators für jede zu applizierende bzw. zu verdampfende Einzeldosis nicht erforderlich.

Weitere zweckmäßige und/oder vorteilhafte Merkmale und Weiterbildungen sowie Verfahrensschritte ergeben sich aus den Unteransprüchen und/oder der Beschreibung und/oder der Zeichnung. Besonders bevorzugte Ausführungsformen sowie das Herstellungserfahren werden anhand der beigefügten Zeichnung näher erläutert. In der Zeichnung zeigt:
- Fig. 1.a) bis 1.b): eine erste Ausführungsform einer erfindungsgemäßen Verdampfereinheit mit hervorstehender und umgebogener Flexkontaktierung,
- Fig. 1aa) bis 1bb).: die Ausführungsformen gemäß Figuren 1a) und 1b), jeweils ergänzt um eine RFID-Antenne und ein weiteres elektronisches Bauteil,
- Fig. 2: eine weitere Ausführungsform einer erfindungsgemäßen Verdampfereinheit,
- Fig. 3.1) bis 3.6): eine mögliche Schrittfolge zum Ausführen eines bevorzugten Verfahrens gemäß der Erfindung anhand eines endlosen Gurtes zur Bearbeitung an unterschiedlichen Fertigungsstationen einer Fertigungslinie,
- Fig. 4: eine aus dem Gurt vereinzelte/getrennte Verdampfereinheit,
- Fig. 5: eine Oberseite eines Abschnitts einer ersten Ausführungsform des Leiterkartenmaterials mit zwei ein Heizorgan aufweisenden Bauplätzen,
- 5.1: die Darstellung gemäß Figur 5 mit einer veränderten Kontaktierung im Bereich der Leiterbahnen,
- Fig. 5a: eine Oberseite eines Abschnitts einer weiteren Ausführungsform des Leiterkartenmaterials mit jeweils zwei ein Heizorgan aufweisenden Bauplätzen und jeweils zwei RFID-Antennen,
- Fig. 6: eine bespielhafte Anordnung vieler Verdampfereinheiten auf einem Leiterkartenabschnitt vor dem Schritt des Vereinzelns von schräg oben,
- Fig. 7: die Anordnung gemäß Figur 6 von schräg unten,
- Fig. 8: eine bevorzugte Ausführungsform einer erfindungsgemäßen Verdampferbaugruppe,
- Fig. 8a: eine weitere bevorzugte Ausführungsform einer erfindungsgemäßen Verdampferbaugruppe,
- Fig. 9: eine bevorzugte Ausführungsform einer erfindungsgemäßen Verdampferkartusche,
- Fig. 10: eine weitere Ausführungsform einer erfindungsgemäßen Verdampferkartusche, und
- Fig. 11: eine bevorzugte Ausführungsform eines erfindungsgemäßen Inhalators.

Die in der Zeichnung dargestellte Verdampfereinheit bzw. die Verdampferbaugruppe und Verdampferkartusche dienen in zu einem Inhalator montierten Zustand zum Inhalieren von mit Wirkstoffen, z.B. Nikotin, angereichertem Dampf und/oder Aerosolen aus Flüssigkeit, sind also im Zusammenhang mit einer E-Zigarette beschrieben. Andere Einsatzzwecke, insbesondere auch ein Einsatz im medizinischen Bereich, sind ausdrücklich mit umfasst, zumal insbesondere die Verdampfereinheiten und die Verdampferbaugruppe einen universellen Einsatz zulassen, da die standardisierte Verdampfereinheit bzw. Verdampferbaugruppe eine Integration/einen Einbau in unterschiedliche Verdampferkartuschen und/oder unterschiedliche Tankformen ermöglicht.

Wie erwähnt, dient das Verfahren zum Herstellen von Verdampfereinheiten 10 als Bestandteil von Inhalatoren 100. Das Verfahren kann manuell oder halbautomatisch ausgeführt werden, indem z.B. nur einzelne Verfahrensschritte automatisiert ausgeführt werden. Bevorzugt ist das Verfahren jedoch für eine vollautomatisierte Ausführung bzw. Herstellung von Verdampfereinheiten ausgebildet.

Das Verfahren zeichnet sich erfindungsgemäß dadurch aus, dass mindestens folgende Schritte in der vorgegebenen Reihenfolge a) bis c) ausgeführt werden, nämlich a) Bereitstellen eines flexiblen Leiterkartenmaterials 11 mit einer Vielzahl von Bauplätzen 12 für einzelne Verdampfereinheiten 10, wobei das flexible Leiterkartenmaterial 11 mindestens mit hinsichtlich Position und/oder Verlauf vorbestimmten Leiterbahnen 13, 14 und/oder Vorstanzungen 15, 16, 24 für jeden Bauplatz 12 wahlweise vorstrukturiert ist oder vorstrukturiert wird, b) Bereitstellen und Platzieren mindestens eines elektrisch mit den Leiterbahnen 13, 14 wahlweise verbundenen oder verbindbaren Heizorgans 17 an dem oder jedem Bauplatz 12, und c) mindestens teilweises Abdecken jedes Bauplatzes 12 mit einem Dichtmaterial 18 zur Bildung einer partiellen Ummantelung 19 für jede gebildete Verdampfereinheit 10, wobei das Dichtmaterial 18 derart aufgebracht wird, dass die aus Dichtmaterial 18 gebildete Ummantelung 19 jedes Heizorgan 17 unter Freihaltung einer Heizfläche 20 mindestens auf einer Oberseite O des flexiblen Leiterkartenmaterials 11 mindestens im Randbereich abdeckt, und mindestens die vom Leiterkartenmaterial 11 und/oder dem Heizorgan 17 wegweisenden Außenflächen der Ummantelung 19 Dichtflächen 21 bilden.

Mit jedem bzw. dem oder jedem Bauplatz 12 ist ausdrücklich auch umfasst, dass zum Beispiel nur jeder zweite Bauplatz 12 oder nur fehlerfreie Bauplätze 12 bearbeitet werden. Es ist also jeder gewünschte bzw. ausgewählte bzw. zu produzierende Bauplatz 12 gemeint. Im Idealfall sind für eine besonders hohe Effizienz tatsächlich ausnahmslos alle Bauplätze 12 zu bearbeiten. Im Schritt a) kann das Bereitstellen des Leiterkartenmaterials 11 auf unterschiedliche Weise erfolgen (siehe hierzu weiter unten). Das Leiterkartenmaterial 11 kann bereits vorstrukturiert bereitgestellt werden oder nach dem Bereitstellen und vor der weiteren Bearbeitung, also im Herstellungsprozess der Verdampfereinheiten 10 noch vorstrukturiert werden. Im Schritt b) kann das Heizorgan 17, z.B. für den Fall, dass das Heizorgan 17 ein Folienheizer ist, bereits verbunden sein, während das Heizorgan 17, z.B. für den Fall, dass das Heizorgan 17 ein Siliziumchip ist, im Herstellungsverfahren noch verbunden werden kann. Das oder jedes Heizorgan 17 kann auf beiden Seiten des Leiterkartenmaterials 11 angeordnet sein, z.B. nur auf der Oberseite oder nur auf der Unterseite, wobei Oberseite und Unterseite letztlich austauschbar sind, da die Zuordnung davon abhängt, wie das Leiterkartenmaterial 11 letztlich gehalten/transportiert bzw. die darauf gebildete Verdampfereinheit 10 letztlich montiert wird, oder auf beiden Seiten mit einer Durchkontaktierung. Im Randbereich bedeutet, dass das Heizorgan 17 umlaufend abgedichtet ist, derart, dass eine Fluidverbindung zwischen der Oberseite O und der Unterseite U des Leiterkartenmaterials 11 benachbart zur Heizfläche 20 ausgeschlossen ist.

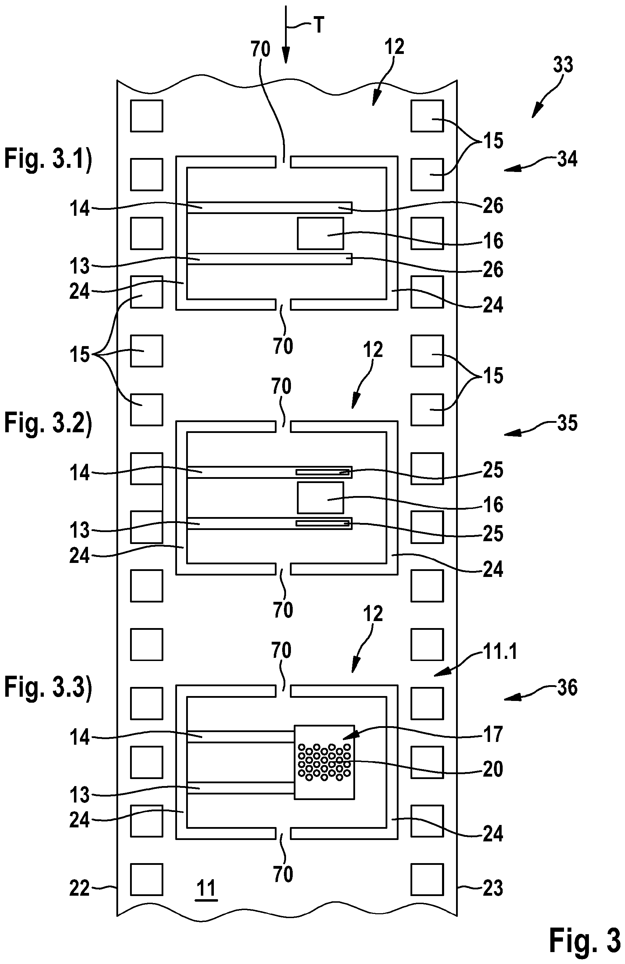

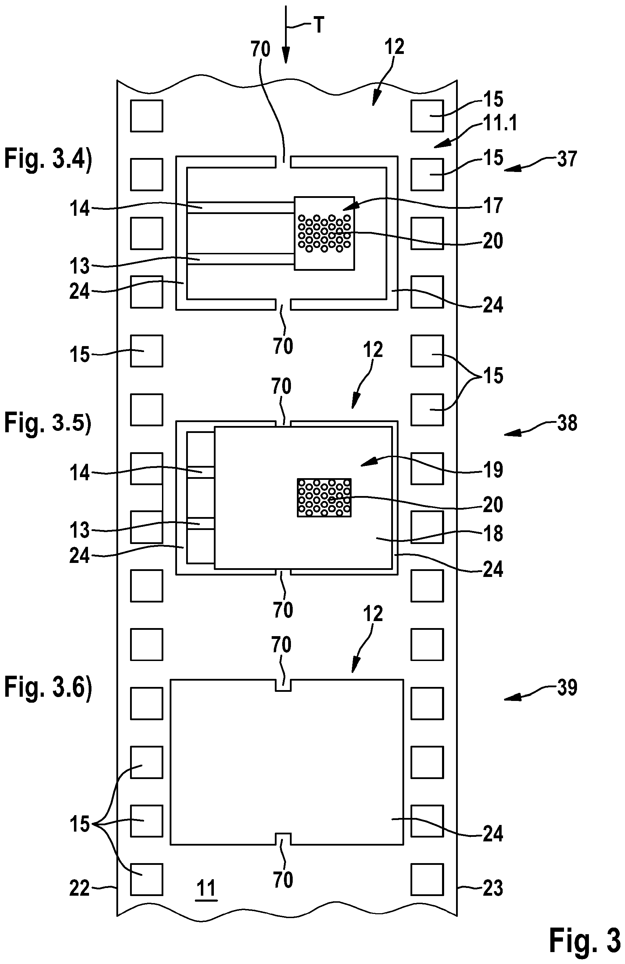

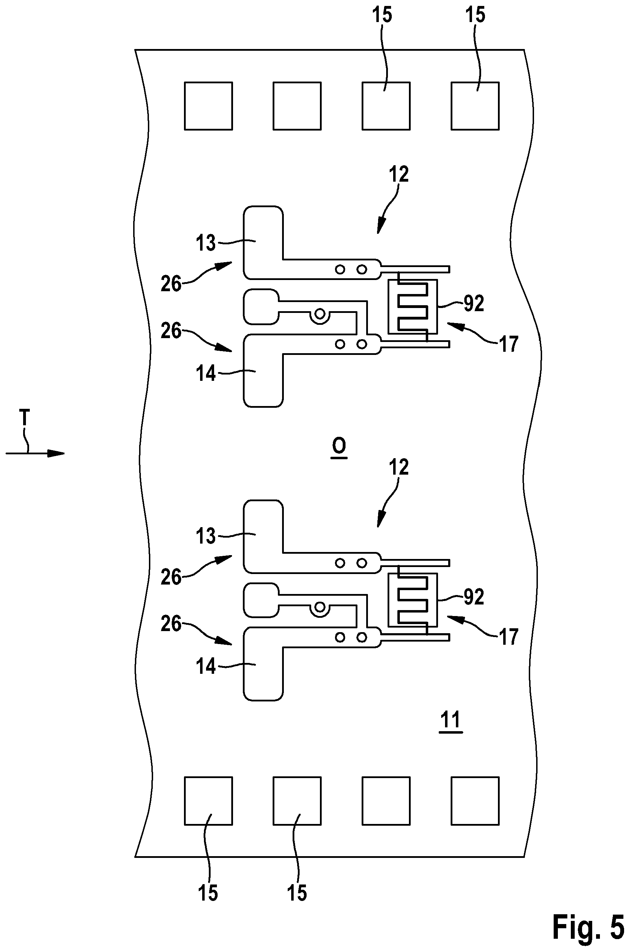

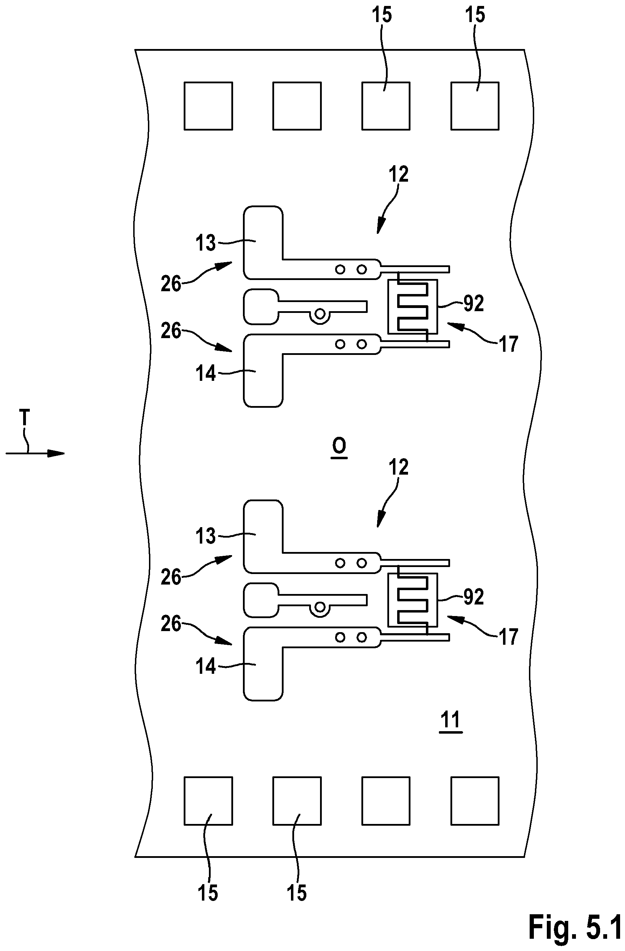

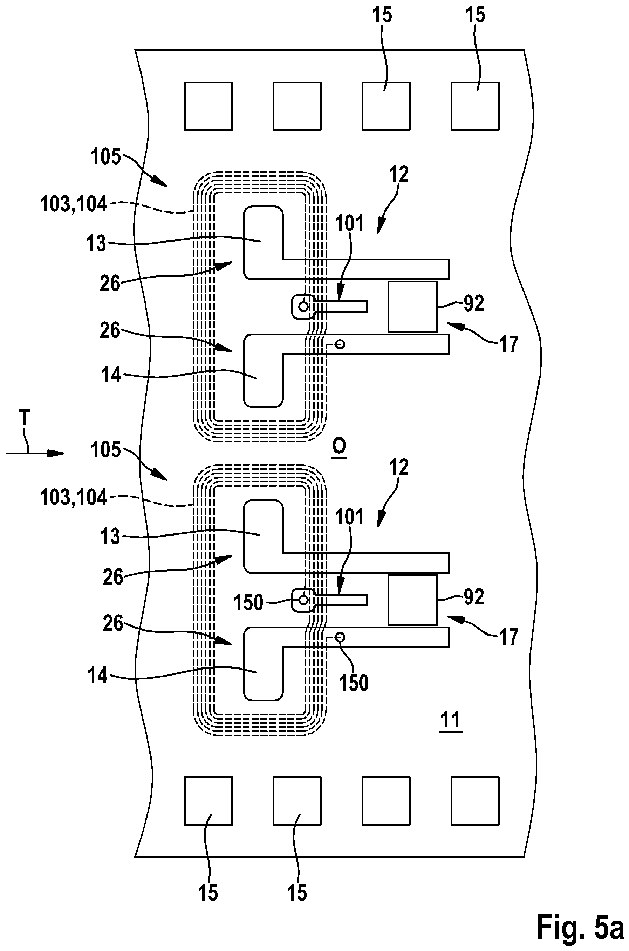

Das flexible Leiterkartenmaterial 11 als Substrat kann z.B. ein einbahniger (und im Folgenden auch als Endlosgurt bezeichneter) Gurt 11.1 (siehe insbesondere Figur 3) oder ein mehrbahniger Endlosgurt sein (siehe z.B. Figuren 5 bis 7), der auf Rollen oder anderen Vorratsgebinden bevorratet ist, und zum weiteren Verarbeiten kontinuierlich oder intermittierend in Transportrichtung T abgerollt bzw. abgewickelt wird. Zum Transport des Endlosgurtes 11.1 weist dieser mindestens an einem Seitenrand 22, vorzugsweise jedoch an beiden Seitenrändern 22, 23, Vorstanzungen 15 auf, in die Transportmittel z.B. in Form von Transportpins, Antriebsrädern oder dergleichen eingreifen können. Das flexible Leiterkartenmaterial 11 als Substrat kann z.B. auch in einen Bauplatz 12 oder mehrere Bauplätze 12 umfassende Abschnitte eingeteilt bzw. getrennt sein, die in Magazinen, Kassetten oder dergleichen bevorratet und zugeführt werden.

Zusätzlich zu den transportbedingten Vorstanzungen/Ausnehmungen 15 kann das Leiterkartenmaterial 11 an jedem Bauplatz 12 einer Verdampfereinheit 10 weitere Vorstanzungen 16, also Ausnehmungen bzw. Öffnungen aufweisen, und zwar insbesondere in solchen Fällen, in denen für die Verdampfereinheiten 10 separat zu bestückende Heizorgane 17 eingesetzt werden. In diesen Fällen dienen die Vorstanzungen 16 an jedem Bauplatz 12 als Durchgangsöffnung 41 zum Ermöglichen einer Verbindung zwischen Vorratstank und Luftströmungskanal über die Verdampfereinheit 10, worauf weiter unten eingegangen wird. Für den Fall, dass das Heizorgan 17 z.B. als Folienheizer integraler Bestandteil des Leiterkartenmaterials 11 ist, kann optional ein Bereich 92 einer Mikrostrukturierung vorgesehen sein, der flüssigkeitspermeabel ausgebildet ist. Weitere Vorstanzungen 24 dienen dazu, das Dichtmaterial 18 von der Oberseite an die Unterseite oder umgekehrt fließen kann. Die Vorstanzungen 24 können auch dazu dienen, die Verdampfereinheit 10 bereits abschnittsweise von dem Leiterkartenmaterial 11 vorzulösen.

Jeder Bauplatz 12 umfasst auch mindestens zwei Leiterbahnen 13, 14 zur elektrischen Kontaktierung des Heizorgans 17. Sowohl die Leiterbahnen 13, 14 als auch die Vorstanzungen 15, 16, die dem Leiterkartenmaterial 11 an dem oder jedem Seitenrand 22, 23 und jedem Bauplatz 12 zugeordnet sind, sind vorstrukturiert bzw. werden vorstrukturiert, derart, dass sie an eine vordefinierte Struktur/ein vordefiniertes Layout von Leiterbahnen 13, 14 und Vorstanzungen 15, 16, 24 passend zu den herzustellenden Verdampfereinheiten 10 angepasst sind. Letztlich kann die Anzahl der Bauplätze 12 auf einem Endlosgurt 11.1 beliebig sein und hinsichtlich Anordnung/Muster variieren, ebenso wie die Layouts der Bauplätze 12 variieren können. Vorzugsweise sind jedoch alle Bauplätze 12 auf einem Endlosgurt 11.1 identisch ausgebildet.

Für den Fall, dass das Leiterkartenmaterial 11 an jedem Bauplatz 12 ein in das Leiterkartenmaterial 11 integriertes Heizorgan 17 zur Bildung einer Heizfläche 20 umfasst (siehe z.B. Figur 5), führt das Ausführen des Schrittes a) zwangsläufig und gleichzeitig auch zum Ausführen des Schrittes b), so dass sich in Kombination mit Schritt c) mindestens ein Zweischrittverfahren ergibt.

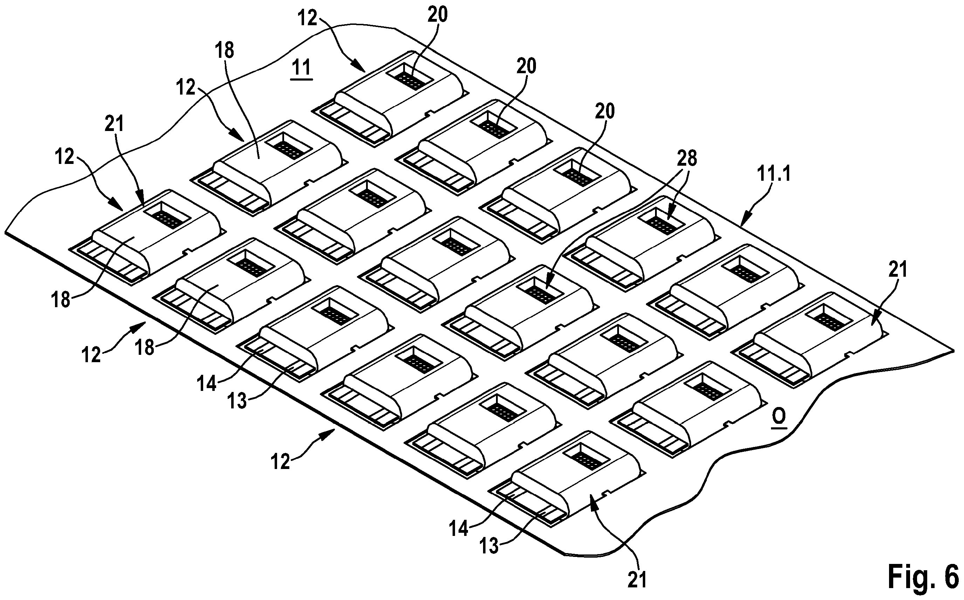

Die Schritte a) und b) können für den Fall, dass das Heizorgan 17 nicht integral mit dem Leiterkartenmaterial 11 ausgebildet ist, nacheinander ausgeführt werden (siehe hierzu weiter unten), so dass sich in Kombination mit Schritt c) mindestens ein Dreischrittverfahren ergibt. Im letztgenannten Fall erfolgt das Bestücken jedes Bauplatzes 12 mit mindestens einem Heizorgan 17 z.B. mittels eines SMT-Bestückungsautomaten von der Oberseite O des Leiterkartenmaterials 11 (siehe Darstellung gemäß Figur 3.3), die auch als Kontaktseite bezeichnet wird. Das mindestens teilweise Abdecken jedes Bauplatzes 12 mit dem Dichtmaterial 18 erfolgt mindestens von der Oberseite O des Leiterkartenmaterials 11. Dafür wird besonders bevorzugt das technisch grundsätzlich bekannte Verfahren "Film-Assisted-Moulding" eingesetzt, derart, dass jedes Heizorgan 17 mindestens im Randbereich umlaufend abgedeckt ist, wobei das Dichtmaterial 18 bevorzugt möglichst dicht an die aktive, eigentliche Heizfläche 20 reicht (siehe Darstellung gemäß Figur 3.5), die frei von Dichtmaterial 18 bleibt, um einen Flüssigkeits-/Dampf-Transport durch das Heizorgan zu gewährleisten. In der Figur 6 ist beispielhaft eine Oberseite O eines Gurtes 11.1 aus flexiblem Leiterkartenmaterial 11 mit mehreren Bauplätzen 12 nach dem Schritt c) gezeigt. Beim teilweisen oder vollständigen Abdecken/Ummanteln/Umspritzen oder dergleichen der Bauplätze 12 werden zunächst Filmfolien, mindestens eine, vorzugsweise zwei, in definierte Formen gelegt, wo sie sich z.B. mittels Vakuum eng an die Innenseite der Formen anlegen, bevor der Endlosgurt 11.1 eingeführt und vorzugsweise Silikone als Dichtmaterial 18 zur Bildung der Ummantelung 19 zugeführt werden. Es ist aber auch denkbar, dass ein geeigneter Kunststoff oder Polyimid verwendet wird. Mit der Ummantelung 19 werden zumindest das Heizorgan 17 teilweise und Teile des Leiterkartenmaterials 18 an jedem Bauplatz 12 abgedeckt bzw. dichtend umschlossen. Das Abdecken jedes Bauplatzes 12 mit dem Dichtmaterial 18 kann aber auch, z.B. für den Fall, dass das Heizorgan 17 auf der Unterseite platziert wird, von der Unterseite U des Leiterkartenmaterials 11 oder von beiden Seiten erfolgen.

Die im Folgenden beschriebenen Verfahrensschritte stellen für sich betrachtet oder in Kombination miteinander bevorzugte Ausführungsformen dar. Es wird ausdrücklich darauf hingewiesen, dass Weiterbildungen und Verfahrensschritte, die in den Ansprüchen und/oder der Beschreibung und/oder der Zeichnung zusammengefasst oder in einer gemeinsamen Ausführungsform beschrieben sind, auch funktional eigenständig das Verfahren und die sich daraus ergebenden Komponenten weiterbilden können.

Vorzugsweise wird im Schritt a) flexibles Leiterkartenmaterial 11 bereitgestellt wird, siehe z.B. Figur 5a, bei dem im Bereich jedes Bauplatzes 12 für eine einzelne Verdampfereinheit 10 mindestens ein Bauplatz 101 für einen RFID-Chip 102 ausgebildet ist, und dass das flexible Leiterkartenmaterial 11 hinsichtlich Position und/oder Verlauf vorbestimmter Leiterbahnen 103, 104 als RFID-Antenne 105 vorstrukturiert ist oder vorstrukturiert wird. In der Figur 5a bildet der Bauplatz 101 einen Anschlussplatz für ein weiteres elektronisches Bauteil, beispielsweise den (RF-)ID-Chip 102. In der Figur 5a ist weiterhin angedeutet, dass die RFID-Antennen 105 auf der Unter-/Rückseite des Leiterkartenmaterials 11 angeordnet bzw. ausgebildet sind, mit entsprechender Durchkontaktierung 150 auf die Oberseite.

Das Leiterkartenmaterial 11 kann bezüglich des zusätzlichen Bauplatzes 101 für den RFID-Chip 102 und dessen elektrische Anbindung oder Funkanbindung unterschiedlich vorstrukturiert sein. Die Leiterbahnen 13, 14 für das Heizorgan 17 können die RFID-Antennen 105 als Spule bilden. Optional bilden jedoch die separaten Leiterbahnen 103, 104 die Spule als RFID-Antenne 105. Es können auch mehrere Leiterbahnen vorstrukturiert sein, um einerseits eine Empfangsantenne (Empfangsspule) und andererseits eine Sendeantenne (Sendespule) zu bilden. Die Kommunikation zwischen RFID-Antenne(n) und RFID-Chip kann elektrisch oder über elektromagnetische Kopplung erfolgen, ebenso wie die Spannungsversorgung des RFID-Chips. Die vorstrukturierten RFID-Antennen können vorzugsweise planparallel zueinander und in geringem Abstand angeordnet und ausgebildet sein. Besonders bevorzugt ist oder wird das flexible Leiterkartenmaterial 11 mindestens mit hinsichtlich Position und/oder Verlauf vorbestimmten Leiterbahnen und/oder Vorstanzungen für jeden Bauplatz 101 zur elektronischen Anbindung mindestens des RFID-Chips 102 wahlweise vorstrukturiert.

Der RFID-Chip 102 kann integraler Bestandteil des vorstrukturierten Leiterkartenmaterials 11 sein. Für den Fall, dass der RFID-Chip 102 ein separates Bauteil ist, wird in einem Schritt k), der dem weiter unten beschriebenen Schritt h) entsprechen kann und der vor, mit oder nach dem Schritt b) erfolgen kann, an jedem Bauplatz 101 ein RFID-Chip 102 bereitgestellt und platziert. Vorzugsweise ist oder wird der elektrisch mit den Leiterbahnen wahlweise verbundene oder verbindbare RFID-Chip 102 bereitgestellt und platziert, wobei der RFID-Chip 102 an jedem Bauplatz 101 mit den die RFID-Antenne 105 bildenden Leiterbahnen 103, 104 wahlweise verbunden oder verbindbar ist, und zwar elektrisch oder über elektromagnetische Kopplung.

Im Übrigen gelten für den RFID-Chip 102 und die RFID-Antenne 105 die zuvor im Zusammenhang mit dem Heizorgan 17 beschriebenen Verfahrensschritte entsprechend. Optional wird im Schritt c) zusätzlich zum teilweise Abdecken jedes Bauplatzes 12 für das Heizorgan 17 auch der Bauplatz 101 für den RFID-Chip 102 mit dem Dichtmaterial 18 unter Bildung von Dichtflächen abgedeckt. Mit anderen Worten wird der RFID-Chip 102 gegenüber der Umgebung durch das Dichtmaterial 18 geschützt.

Wie bereits angedeutet, kann das Bereitstellen des flexiblen Leiterkartenmaterials 11 in Schritt a) wahlweise durch Abrollen des auf Rollen gelagerten bzw. bevorrateten Leiterkartenmaterials 11 oder durch Zuführen von in Magazinen oder dergleichen bevorrateten Abschnitten des Leiterkartenmaterials 11 erfolgen.

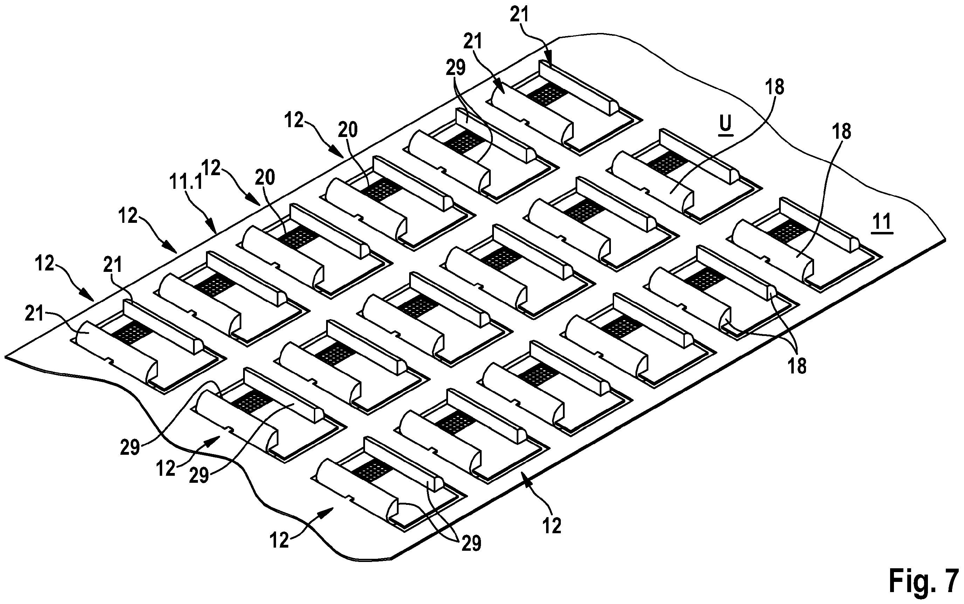

Vorzugsweise wird im Schritt c) jeder Bauplatz 12, 101 mindestens teilweise mit Dichtmaterial 18 umschlossen, derart, dass zusätzlich sowohl auf der Oberseite O als auch auf einer der Oberseite O gegenüberliegenden Unterseite U des flexiblen Leiterkartenmaterials 11 an den Außenflächen der Ummantelung 19 Dichtflächen 21 für eine frei von zusätzlichen Dichtmitteln bestehende Verbindung zu den die Verdampfereinheit 10 umgebenden Komponenten des Inhalators 100 (hierzu weiter unten) ausgebildet werden. Mit anderen Worten erfolgt das Umschließen jedes Bauplatzes 12, 101 von beiden Seiten des Endlosgurtes 11.1, z.B. durch Umspritzen von der Oberseite O und der Unterseite U oder durch Umspritzen ausschließlich von der Oberseite O, wobei das Dichtmaterial 18 durch Vorstanzungen 24 an jedem Bauplatz 12, 101 auf die Unterseite U gelangt. Die Dichtflächen 21 sind bevorzugt teilweise oder vollständig definiert und reproduzierbar ausgebildet. In der Figur 7 ist beispielhaft eine Unterseite U eines Endlosgurtes 11.1 aus flexiblem Leiterkartenmaterial 11 mit mehreren Bauplätzen 12 nach dem Schritt c) gezeigt. Die Vorstanzungen 24 dienen weiterhin dazu, jede Verdampfereinheit 10 möglichst einfach vom Leiterkartenmaterial 11 trennen zu können. Mit Ausnahme von zwei Verbindungsstegen 70 sind die Vorstanzungen 24 vorzugsweise umlaufend um jeden Bauplatz 12, 101 ausgebildet. Es können selbstverständlich auch nur ein Steg 70 oder mehr als zwei Stege 70 vorgesehen sein. Auf Vorstanzungen 24 für das spätere Heraustrennen kann jedoch auch ganz oder teilweise verzichtet werden. In diesem Fall sind nur Vorstanzungen 24 vorgesehen, wenn Dichtmaterial 18 das Substrat umfließen soll.

Für den weiter oben bereits angedeuteten Fall, dass das Heizorgan 17 nicht integraler Bestandteil des Leiterkartenmaterials 11 ist, sondern das Leiterkartenmaterial 11 mit jedem Heizorgan 17 separat zu bestücken ist, umfasst der Schritt b) als Schritt b₁) das Auftragen von elektrischem Kontaktierungsmaterial 25 mindestens auf Kontaktflächen 26 der Leiterbahnen 13, 14 (siehe Darstellung gemäß Figur 3.2) an jedem Bauplatz 12 auf der Oberseite O des flexiblen Leiterkartenmaterials 11, als Schritt b₂) das Platzieren des mindestens einen Heizorgans 17 im Bereich jedes Bauplatzes 12, derart, dass eine die Oberseite O mit der Unterseite U des Leiterkartenmaterials 11 verbindende Vorstanzung 16, die später die Durchgangsöffnung der Verdampfereinheit 10 bildet, vollständig durch das oder jedes Heizorgan 17 abgedeckt ist (siehe Darstellung gemäß Figur 3.3), und als Schritt b₃) das Ausbilden einer elektrischen Verbindung zwischen Heizorgan 17 und Leiterbahnen 13, 14 an jedem Bauplatz 12. Nach der Bereitstellung des mit Leiterbahnen 13, 14 und Vorstanzungen 15, 16, 24 vorkonfektionierten Endlosgurtes (siehe Darstellung gemäß Figur 3.1) an einer ersten Fertigungsstation wird an einer zweiten Fertigungsstation z.B. Sinterpaste auf die Kontaktflächen 26 aufgetragen. Anschließend wird jedes Heizorgan 17 von einem Bestückungsautomaten aufgenommen, positioniert und auf den mit Sinterpaste benetzten Kontaktflächen 26 abgesetzt/platziert, so dass die die Durchlassöffnung bildende Vorstanzung 16 vollständig vom Heizorgan 17 überdeckt ist. Durch Ausüben eines Sintervorgangs z.B. in einer Sinterpresse, wird eine dauerhafte elektrische Verbindung zwischen Heizorgan 17 und Leiterbahnen 13, 14 erzeugt, wodurch das Heizorgan 17 zusätzlich auf dem Leiterkartenmaterial 11 fixiert ist. Ganz besonders bevorzugt wird jedes Heizorgan 17 durch eine Direktkontaktierung mittels Silber-Sintern elektrisch mit den Leiterbahnen 13, 14 des flexiblen Leiterkartenmaterials 11 verbunden. Die Schritte b₁) und b₂) können auch miteinander verknüpft werden, indem z.B. das Bestücken des Heizorgans 17 durch eine mit Kontaktierungsmaterial 25 versehene Folie oder dergleichen erfolgt, so dass die Heizorgane 17 beim Durchdrücken und Platzieren auf den Kontaktflächen 26 das Kontaktierungsmaterial 25 quasi "mitbringen". Alternativ ist jedes andere oben beschriebene Verfahren zur Herstellung einer elektrischen Verbindung zwischen den Leiterbahnen 13, 14 und dem Heizorgan 17 geeignet. Letztlich kann auch das Heizorgan 17 mit Kontaktierungsmaterial 25 vorkonfektioniert sein, beispielsweise, indem ein Siliziumchip auf einem Wafer bereits mit Kontaktierungsmaterial 25 laminiert ist.

Für den Fall, dass der RFID-Chip 102 separat zu bestücken ist, umfasst der Schritt k) als Schritt k₁) das Auftragen von elektrischem Kontaktierungsmaterial 25 mindestens auf Kontaktflächen der Leiterbahnen an jedem Bauplatz 101 auf der Oberseite O des flexiblen Leiterkartenmaterials 11, als Schritt k₂) das Platzieren des mindestens einen RFID-Chips 102 im Bereich jedes Bauplatzes 101, und als Schritt k₃) das Ausbilden einer elektrischen Verbindung zwischen RFID-Chip 102 und Leiterbahnen an jedem Bauplatz 101. Wie weiter oben bereits beschrieben, kann das elektrische Kontaktierungsmaterial im Schritt k₁) optional auch direkt auf die Kontakte des RFID-Chips aufgebracht werden, wobei der so präparierte RFID-Chip dann auf den Bauplatz 101 platziert wird.

Vorstehend ist das Verfahren für den Fall beschrieben, dass das Heizorgan 17 auf der Oberseite des Leiterkartenmaterials 11 platziert und verbunden wird. Optional und entsprechend ist auch eine Platzierung und Verbindung auf der Unterseite möglich. Es besteht auch die Möglichkeit, dass das Heizorgan 17 auf der Oberseite platziert ist, wobei die elektrischen Kontakte, also z.B. die Leiterbahnen 13, 14 auf der Unterseite angeordnet sind. Vorstanzungen 24 im Bereich der elektrischen Kontakte des Heizorgans 17 verbinden die Oberseite und die Unterseite, so dass Kontaktierungsmaterial 25 im Bereich der Vorstanzungen 24 aufgebracht wird, das nach dem Schritt b₃) eine Verbindung zwischen Heizorgan 17 auf der Oberseite und Leiterbahnen 13, 14 auf der Unterseite herstellt. Entsprechendes gilt für jeden RFID-Chip 102.

Die im Folgenden genannte Aufzählung der Schritte d) bis h) und k) stellt keine zwingende Reihenfolge der Schritte dar. Vielmehr können die Schritte d) bis h) und k) quasi beliebig in ihrer Reihenfolge ausgeführt werden. Besonders bevorzugt wird der Schritt k) direkt vor oder nach dem Schritt b) ausgeführt.

Nach dem Schritt c) kann der Endlosgurt 11.1 beispielsweise wieder aufgerollt werden, um für die Herstellung von Verdampfereinheiten 10 einen Rolle-zu-Rolle-Prozess nutzen zu können. Das bedeutet, dass initial im Schritt a) eine Rolle mit aufgerolltem flexiblem Leiterkartenmaterial 11 bereitgestellt wird. Anschließend wird in den Schritten b) und c) eine Vielzahl von Verdampfereinheiten 10 darauf abgebildet. Schließlich kann das flexible Leiterkartenmaterial 11 in einem Schritt e) zur weiteren Verwendung, beispielsweise zum Transport, zum Bevorraten oder dergleichen wieder/erneut aufgerollt werden. Wenn Abschnitte von Leiterkartenmaterial 11 verarbeitet werden, können diese z.B. in dem Schritt e) wieder einem Magazin oder dergleichen zur weiteren Verwendung zugeführt werden.

Beim Abdecken bzw. Umschließen der Bauplätze 12 mit dem Dichtmaterial 18 entstehen prozessbedingt so genannte Angussstellen, also Materialstege, die sich beim Gießen, Spritzen oder bei anderweitiger Verarbeitung des Dichtmaterials 18 ergeben. Vorzugsweise nach dem Schritt c) werden die beim Abdecken bzw. Umschließen der Bauplätze mit Dichtmaterial 18 entstehenden Angussabschnitte des Dichtmaterials 18 in einem Schritt d) entfernt. Das Entfernen kann auch später, z.B. beim Heraustrennen einzelner Verdampfereinheiten 10 erfolgen. Das erneute Aufrollen kann, wie erwähnt, z.B. nach dem Schritt c) und optional nachfolgend nach jedem weiteren Schritt erfolgen. Das Aufrollen kann optional sogar bereits nach dem Schritt b) erfolgen. Entsprechend kann das mit der Vielzahl fertiggestellter, dichtender Verdampfereinheiten 10 versehene Leiterkartenmaterial 11 frei von Angussabschnitten zur weiteren Verwendung vorzugsweise erstmals oder wieder in dem Schritt e) auf eine Rolle aufgerollt werden. Entsprechendes gilt bei einer Verwendung von Abschnitten des Leiterkartenmaterials 11 in Magazinen oder dergleichen.

Die hergestellten, dichtenden, also mit Dichtflächen 21 versehenen, Verdampfereinheiten 10, die in Kombination/durch Montage mit anderen Komponenten des Inhalators 100 ohne zusätzliche Dichtmittel dichtend sind, werden zur weiteren Verwendung in einem Schritt f) von dem flexiblen Leiterkartenmaterial 11 vereinzelt. Zum Vereinzeln kann das aufgerollte und mit Verdampfereinheiten 10 versehene Leiterkartenmaterial 11 zu einem späteren Zeitpunkt wieder abgerollt werden, beispielsweise an einem Montageplatz für Verdampfereinheiten 10. Das Vereinzeln kann z.B. durch Stanzen, Schneiden oder dergleichen erfolgen. Das Vereinzeln kann auch unmittelbar nach dem Schritt d) erfolgen, um z.B. Tray- oder Bulkware herzustellen.

Zur Vervollständigung jeder Verdampfereinheit 10 wird in einem Schritt g) auf der Oberseite O des flexiblen Leiterkartenmaterials 11 auf wenigstens einem Teil der freien Heizflächen 20 der Heizorgane 17 an jedem Bauplatz 12 mindestens ein Dochtorgan 27 platziert. Das Dochtorgan 27 kann z.B. als Block aus Keramik oder als Glasfaser- oder Baumwollpad oder anderweitig einstückig ausgebildet sein und z.B. zusammen mit dem Schritt b) oder nach dem Schritt b) in beliebiger Schrittfolge platziert werden. Das gemeinsame Bestücken im Schritt b) ist besonders dann bevorzugt, wenn das Dochtorgan 27 und das Heizorgan 17 einen Verbund bilden. Das Dochtorgan 27 kann aber auch nach dem Schritt c) oder nach dem Schritt d) oder auch nach dem Schritt f) platziert werden. In einer anderen Variante, in der das Dochtorgan 27 z.B. ein granulares Dochtorgan 27 ist, kann das Granulat z.B. nach dem Vereinzeln in eine in der Ummantelung 19 gebildeten Ausnehmung 28, in der die Heizfläche 20 des Heizorgans 17 frei liegt, geschüttet werden. Durch eine Kombination/Montage mit einer weiteren Komponente des Inhalators 100 (siehe hierzu weiter unten) kann das Dochtorgan 27 dann z.B. durch Kleben oder andere Verbindungstechniken fixiert werden. Das Dochtorgan 27 kann in anderen Ausführungsformen auch auf einer dem Heizorgan 17 gegenüberliegenden Seite platziert und/oder fixiert werden. Weder das Heizorgan 17 noch das Dochtorgan 27 müssen zwingend auf einer Seite angeordnet sein und insbesondere auch nicht zwingend auf der Oberseite.

Optional kann in einem Schritt h), der dem Schritt k) entsprechen kann, vorzugsweise zusammen mit dem Schritt b₂) bzw. k₂) mindestens ein zusätzliches elektronisches Bauteil im Bereich jedes Bauplatzes 12 platziert werden. Der Schritt h) kann aber auch vor oder nach dem Schritt b₂) bzw. k₂) erfolgen. Vor dem Schritt h), vorzugsweise zusammen mit Schritt b₁ und k₁), wird Kontaktierungsmaterial 25 auf Kontaktflächen 26 der Leiterbahnen 13, 14 an jedem Bauplatz 12 auf der Oberseite O und/oder der Unterseite U des flexiblen Leiterkartenmaterials 11 aufgebracht, und nach dem Schritt h), vorzugsweise zusammen mit Schritt b₃) bzw. k₃), wird eine elektrische Verbindung zwischen dem oder jedem zusätzlichen elektronischen Bauteil und Leiterbahnen 13, 14 an jedem Bauplatz 12 ausgebildet. Dabei kann es sich um die Leiterbahnen 13, 14 des Heizorgans 17 handeln. Die elektronischen Bauteile können aber auch an/auf eigenen Leiterbahnen elektrisch kontaktiert sein. Die elektronischen Bauteile können auf der Seite des Heizorgans 17 und/oder auf der gegenüberliegenden Seite platziert sein. Das oder jedes zusätzliche elektronische Bauteil, z.B. ID-Chips, Sensoren oder andere elektronische Bauteile können teilweise oder vollständig vom Dichtmaterial 18 abgedeckt bzw. umschlossen werden. Dies kann z.B. im Schritt c) erfolgen. Es besteht auch die Möglichkeit, dass die elektronischen Bauteile in einem separaten Schritt durch dasselbe Dichtmaterial 18 oder ein anderes Material ganz oder teilweise ummantelt werden.

Jedes Heizorgan 17 und/oder jeder RFID-Chip 102 und/oder jedes elektronische Bauteil, insbesondere jeder ID-Chip, wird elektrisch mit den Leiterbahnen 13, 14, oder anderen Kontaktierungsflächen des Leiterkartenmaterials 11 verbunden durch Einsatz eines oder mehrerer Verfahren aus der (nicht abschließenden) Liste: Direktkontaktierung mittels Silber-Sintern, eutektisch Bonden, leitfähig Kleben, anisotrop leitfähig Kleben, KlettWelding, KlettSintering, Löten, Schweißen. Insbesondere die RFID-Chips und andere elektronische Komponenten können optional auch mittels Wirebonding mit den Leiterbahnen 13, 14 des Leiterkartenmaterials 11 verbunden werden. Dazu werden die elektronischen Bauteile (RFID-Chip etc.) mit einem nicht leitfähigen Klebstoff auf dem Leiterkartenmaterial 11 fixiert und dann verbunden.

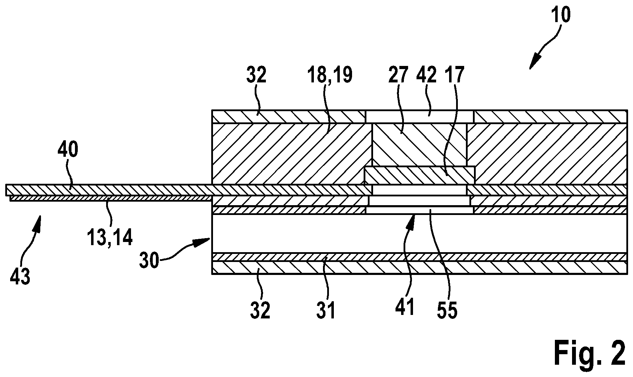

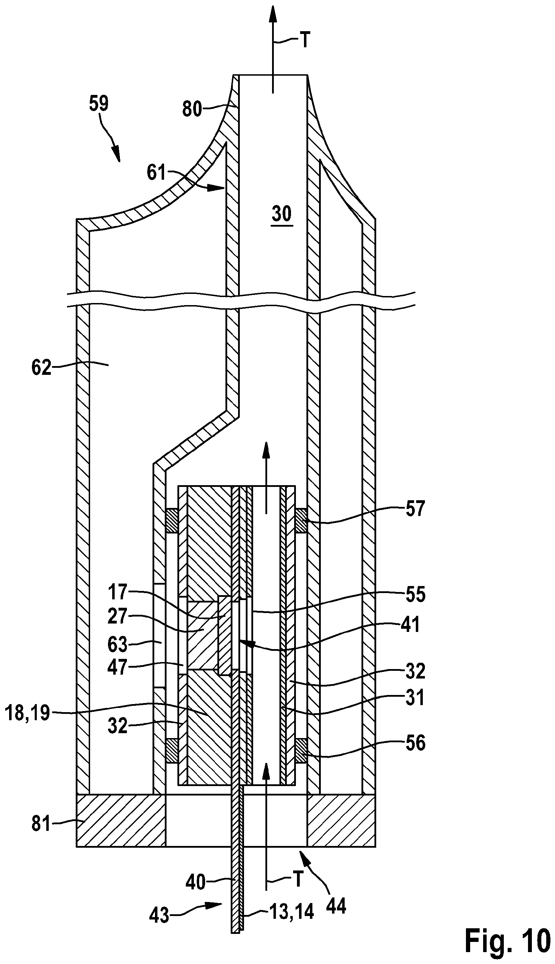

In einer Weiterbildung des Verfahrens können z.B. im Schritt c) beim Abdecken bzw. Umschließen jedes Bauplatzes 12 aus und/oder mit dem Dichtmaterial 18 auf der Unterseite U des Leiterkartenmaterials 11 an jedem Bauplatz 12 mindestens, vorzugsweise stegartige, Abschnitte 29 eines Luftströmungskanals 30 gebildet werden (siehe z.B. Figur 7). Die Abschnitte 29 bilden dabei einen Teil der Wandung des zu bildenden Luftströmungskanals 30. Das Anformen von Teilen des Luftströmungskanals 30 oder eines vollständig ausgebildeten Luftströmungskanals 30 kann auch in einem separaten Schritt mit einem vom Dichtmaterial 18 abweichenden Material erfolgen. In einer weiteren Ausführung kann an jedem Bauplatz 12 ein rohrförmiger, vorkonfektionierter Abschnitt 31 (auch Rohrabschnitt 31 genannt) als Teil eines Luftströmungskanals 30 auf der Unterseite U des Leiterkartenmaterials 11 platziert und mittels zusätzlichem Dichtmaterial 32 umschlossen und damit fixiert werden (siehe Figuren 2 und 10). Wie erwähnt, können vor/während/nach dem Schritt c) auf der Unterseite U des flexiblen Leiterkartenmaterials 11 Dicht- und/oder Luftströmungskanalabschnitte gebildet werden. Die Dicht- und/oder Strömungsfunktion der Verdampfereinheit 10 wird im Zusammenhang mit der Montage weiter unten näher beschrieben.

Mindestens die Schritte a) bis c) werden nacheinander jeweils an einer eigenen oder der gleichen Fertigungsstation einer Fertigungslinie 33 ausgeführt, derart, dass bei jedem Schritt an jeder Fertigungsstation 34 bis 39 eine Vielzahl von Bauplätzen 12, 101 zeitgleich bearbeitet wird bzw. werden kann. Beispielsweise besteht die Option, dass das Sintern zum Herstellen der elektrischen Verbindung und das Umschließen mit Dichtmaterial 18 in/an einer Fertigungsstation erfolgt. Zeitgleich bedeutet im Sinne der Erfindung, dass an mehreren Bauplätzen 12, 101 die gleichen Schritte durchgeführt werden oder das an mehreren Bauplätzen 12, 101 mehrere/verschiedene Schritte durchgeführt werden. Besonders bevorzugt erfolgt jedoch der gesamte Herstellungsprozess der Verdampfereinheiten 10 in einer gemeinsamen Fertigungslinie 33 mit mehreren Fertigungsstationen 34 bis 39. Schematisch ist eine beispielhafte Fertigungslinie 33 in Figur 3 angedeutet. An einer Fertigungsstation 34 (Darstellung gemäß Figur 3.1) wird in einem Schritt a) die oder jede Rolle mit aufgerollten Leiterkartenmaterialien 11 bevorratet und zur Abwicklung oder ggf. auch zum aktiven Abrollen bereitgestellt. An einer Fertigungsstation 35, beispielsweise einem Siebdruckautomaten, wird in einem Schritt b₁) bzw. k₁) elektrisches Kontaktierungsmaterial 25 aufgebracht (Darstellung gemäß Figur 3.2). An einer Fertigungsstation 36, beispielsweise einem SMT-Bestückungsautomaten, wird in einem Schritt b₂) bzw. k₂) das oder jedes Heizorgan 17 bzw. der oder jeder RFID-Chip 102 aufgenommen und platziert (Darstellung gemäß Figur 3.3). An einer Fertigungsstation 37, beispielsweise einer Sinterpresse, wird in einem Schritt b₃) bzw. k₃) die direkte elektrische Kontaktierung ausgebildet (Darstellung gemäß Figur 3.4). An einer Fertigungsstation 38, beispielsweise einem Film-Assisted-Moulding-Automaten, wird in einem Schritt c) jeder Bauplatz 12, 101 mindestens teilweise mit Dichtmaterial 18 abgedeckt bzw. umschlossen (Darstellung gemäß Figur 3.5). An einer Fertigungsstation 39, beispielsweise einem Stanzautomaten, werden in einem Schritt f) die Verdampfereinheiten 10 vereinzelt, indem sie von dem Leiterkartenmaterial 11 getrennt/gelöst werden (Darstellung gemäß Figur 3.6). Zum Herstellen der Verdampfereinheiten 10 wird das flexible und vorzugsweise aus Polyimid bestehende, vorstrukturierte Leiterkartenmaterial 11 in einem Beispiel als Endlosstrang von einer Vorratsrolle abgewickelt und kontinuierlich oder intermittierend durch die mehrere Fertigungsstationen 34 bis 39 umfassende Fertigungslinie 33 transportiert. In anderen Beispielen können Abschnitte des Leiterkartenmaterials 11 z.B. in Magazinen durch die Fertigungslinie transportiert werden. Alternativ sind aber auch alle weiter oben beschriebenen Kontaktierungsverfahren während des Herstellungsprozesses verwendbar. Es können auch zwei oder mehr als zwei Fertigungslinien 33 vorgesehen sein.

Im Folgenden werden rein beispielhaft bevorzugte Prozessreihenfolgen/Schrittfolgen beschrieben. Das Leiterkartenmaterial 11 wird auf Rollen bereitgestellt. An einer ersten Fertigungslinie 33 wird das Leiterkartenmaterial 11 abgewickelt. Mittels Schablonendruck wird das Leiterkartenmaterial 11 an den Positionen, an denen später die Heizorgane 17 platziert werden, mit einer Sinterpaste bedruckt. Nach dem Inspizieren des Druckbilds werden die Heizorgane 17 mittels eines SMT-Placers, also einer pick-andplace-Vorrichtung, platziert. Anschließend wird das Leiterkartenmaterial 11 mit einer heißen Platte von unten getrocknet. Das Sintern erfolgt mit heißen Stempeln von oben auf derselben Platte. Abschließend wird das bestückte Leiterkartenmaterial 11 wieder aufgerollt.

An einer weiteren Fertigungslinie 33 mit entsprechendem Aufbau oder derselben Fertigungslinie 33, ggf. mit angepassten Programmen zum Bedrucken und Platzieren mindestens eines (RF-)ID-Chips, kann die vorstehende Schrittfolge wiederholt werden, so dass dann mit Heizorganen 17 und (RF-)ID-Chips 102 bestücktes Leiterkartenmaterial 11 aufgerollt bereitgestellt ist.

Das derart vorbereitet und bestückte Leiterkartenmaterial 11 kann dann an einer weiteren, aufgrund geringerer Kapazitäten vorzugsweise separaten, Fertigungslinie weiterverarbeitet werden, wobei die oder jede zusätzliche Fertigungslinie als FAM-Linie (Film-Assisted-Molding) ausgebildet ist. In der FAM-Linie bzw. FAM-Vorrichtung kann das vorkonfektionierte Leiterkartenmaterial 11 abgerollt werden. Nach dem Abrollen werden die Anbindungsplätze/Bauplätze einschließlich der Heizorgane 17 und der (RF-)ID-Chips 102 mit Silikon ummantelt. Nach dem Abtrennen der Angussreste wird das Leiterkartenmaterial 11 wieder aufgerollt.

Diese Prozessfolge stellt nur ein ausgewähltes Beispiel dar, das nahezu beliebig variiert werden kann.

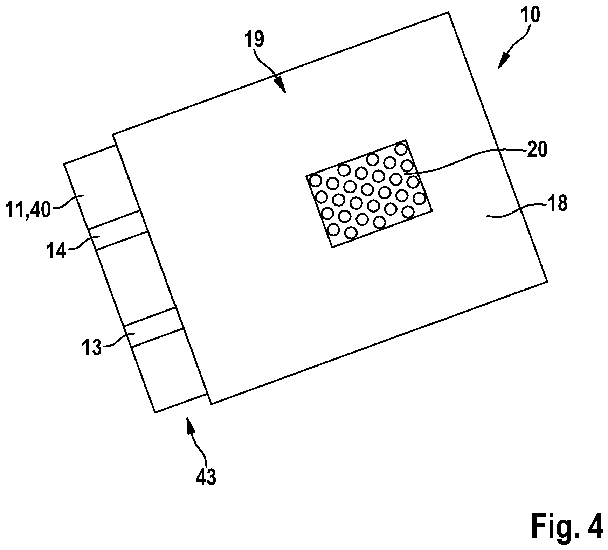

Mit dem Verfahren werden vereinzelte Verdampfereinheiten 10 hergestellt (siehe z.B. Figur 4). Die erfindungsgemäßen Verdampfereinheiten 10 als Bestandteil von Inhalatoren 100 zeichnen sich dadurch aus, dass sie folgende Komponenten aufweisen: einen Leiterkartenabschnitt 40 aus flexiblem Leiterkartenmaterial 11, wobei der Leiterkartenabschnitt 40 mindestens zwei Leiterbahnen 13, 14 und eine Durchlassöffnung 41 umfasst, die eine Oberseite O des Leiterkartenabschnitts 40 mit einer Unterseite U des Leiterkartenabschnitts 40 verbindet, Heizorgan 17, das die Durchlassöffnung 41 von der Oberseite O her vollständig abdeckt und in elektrischem Kontakt zu den Leiterbahnen 13, 14 steht, sowie eine aus Dichtmaterial 18 bestehende Ummantelung 19, die den Leiterkartenabschnitt 40 und das Heizorgan 17 unter Freihaltung einer Heizfläche 20 mindestens teilweise abdeckt und nach außen hin mit Dichtflächen 21 versehen ist. Eine schematische Darstellung ist der Figur 4 zu entnehmen. Der Leiterkartenabschnitt 40 kann anstelle der Durchlassöffnung 41 jedoch ein integrales Heizorgan 17 umfassen, das einen Bereich 92 einer Mikrostrukturierung aufweist und elektrisch mit den Leiterbahnen 13, 14 verbunden ist (siehe Figur 5). Technisch gleichwirkend ist z.B. auch ein Heizorgan 17, das die Durchlassöffnung 41 von der Unterseite U vollständig abdeckt und mit den Leiterbahnen 13, 14 in elektrischem Kontakt steht.

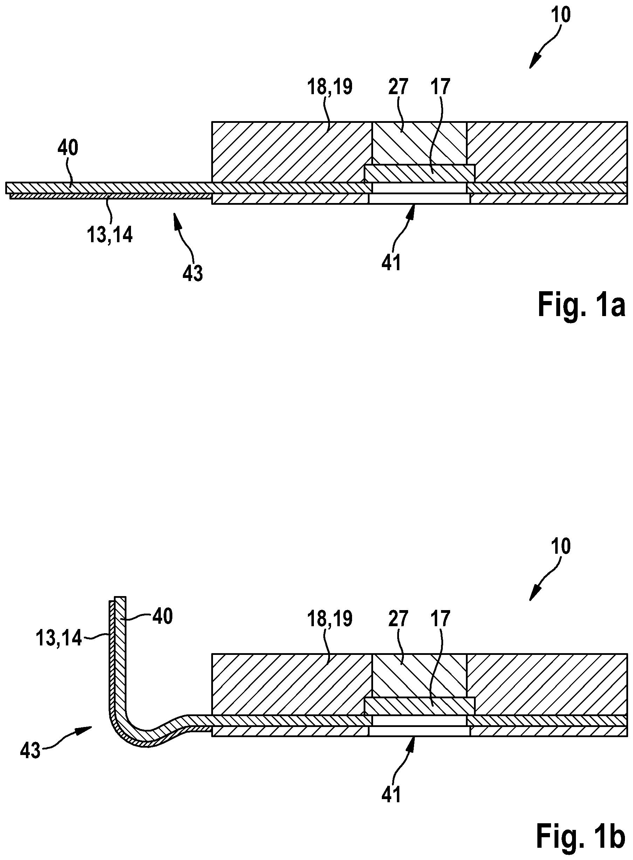

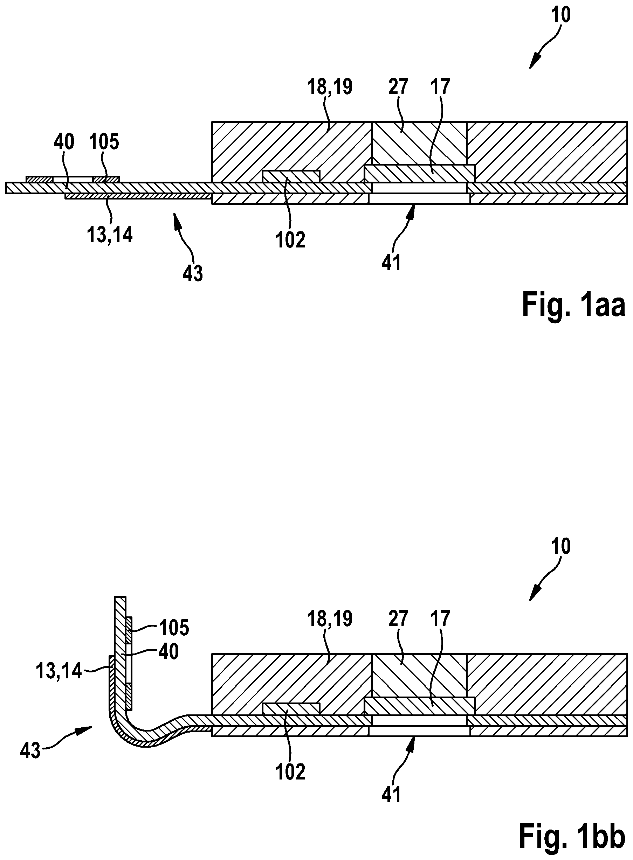

In den Figuren 1a), 1b) und 2 sind vergrößerte Darstellungen von bevorzugten Ausführungsformen von Verdampfereinheiten 10 im Schnitt gezeigt, bei denen auf der Heizfläche 20 ein Dochtorgan 27 platziert ist. In der Figur 1 ist eine Basisvariante ohne Luftströmungskanal 30 dargestellt. Über die Durchlassöffnung 41 und das flüssigkeitspermeable Heizorgan 17 ist der Durchtritt von erzeugtem Dampf in Richtung des Strömungskanals 30 gewährleistet. In der Figur 2 ist auf der Unterseite U des Leiterkartenabschnitts 40 ein vorkonfektionierter Rohrabschnitt 31 zur Bildung des Luftströmungskanals 30 mit der Verdampfereinheit 10 ausgebildet, wobei der Rohrabschnitt 31 mittels des zusätzlichen Dichtmaterials 32 mit der Verdampfereinheit 10 verbunden ist. Oberhalb des Dochtorgans 27 ist eine Öffnung 42 im Dichtmaterial 32 vorgesehen, so dass das Dochtorgan 27 in Richtung eines Vorratstanks frei liegt, um eine Fluidverbindung zum Vorratstank herzustellen. Der Rohrabschnitt 31 weist im Bereich des Heizorgans 17 und der Durchlassöffnung 41 eine Öffnung 55 auf, um einen Austritt der verdampften Flüssigkeit in den Luftströmungskanal 30 zu ermöglichen. Alternativ kann der Luftströmungskanal 30 auch teilweise oder ganz aus Dichtmaterial 18 gebildet sein. Bei einer teilweisen Ausbildung kann die Verdampfereinheit 10 z.B. die in Figur 7 gezeigten stegartigen Abschnitte 29 aufweisen. Diese stegartigen Abschnitte 29 bilden eine Teilwandung eines in Umfangsrichtung nicht vollständig geschlossenen Luftströmungskanals 30, der mit Wandungen bzw. Flächen oder dergleichen anderer Komponenten (wie z.B. der inneren Geometrie des Adaptersteckers, einer Verdampferkartusche oder dergleichen) zusammenwirkt und so einen in Umfangsrichtung vollständig geschlossenen Luftströmungskanal 30 bildet.

In allen hier beschriebenen Ausführungsformen einer Verdampfereinheit 10 kann die elektrische Kontaktierung, d.h. elektrische Verbindung des Heizorgans 17 mit den Leiterbahnen 13, 14 direkt oder indirekt erfolgen. Bei einer direkten Kontaktierung ist wenigstens ein Teil der Leiterbahnen 13, 14, der mit dem Heizorgan 17 elektrisch kontaktiert werden soll oder kontaktiert ist, auf der gleichen Seite des Leiterkartenabschnitts 40 wie das Heizorgan 17 angeordnet. Die elektrische Kontaktierung erfolgt in diesem Fall über die hier im Zusammenhang mit der Erfindung beschriebenen Verfahren. Bei der indirekten Kontaktierung ist der Teil der Leiterbahnen 13, 14, der mit dem Heizorgan 17 elektrisch kontaktiert werden soll oder kontaktiert ist, auf der Seite des Leiterkartenabschnitts 40 angeordnet, die der Seite, an dem das Heizorgan 17 angeordnet ist angeordnet werden soll, gegenüberliegt. In diesem Fall erfolgt eine elektrische Kontaktierung, d.h. Verbindung der elektrischen Kontakte des Heizorgans 17 mit den Leiterbahnen 13, 14 durch den Leiterkartenabschnitt 40 hindurch. Entsprechend sind diese Kontaktierungen in den dargestellten Ausführungsbeispielen nicht gezeigt bzw. nicht sichtbar.

Zur Herstellung der Kontaktierung kann eine oder mehrere Öffnungen in dem Leiterkartenabschnitt 40 vorgesehen sein, in dem oder in denen elektrisch leitfähiges Material angeordnet wird, das die elektrische Verbindung zwischen dem Heizorgan 17 und den Leiterbahnen 13, 14 herstellt. Alternativ können bereits bei der Vorkonfektionierung des Leiterkartenmaterials 11 von der einen zur anderen Seite, d.h. von der Oberseite O zur Unterseite U des Leiterkartenmaterials 11, durchgehende elektrische Verbindungen vorgesehen sein oder werden, so dass auf der den Leiterbahnen 13, 14 gegenüberliegenden Seite Kontaktpunkte vorhanden sind, mit denen das Heizorgan 17 elektrisch verbunden werden kann. Auch in diesem Fall erfolgt die elektrische Kontaktierung über die hier im Zusammenhang mit der Erfindung beschriebenen Verfahren.

In einer bevorzugten Ausführungsform umfasst der Leiterkartenabschnitt 40 mindestens einen RFID-Chip 102 und mindestens eine RFID-Antenne 105. Die RFID-Antenne 105 kann durch Windungen des Heizorgans 17 gebildet sein. Vorzugsweise ist die oder jede RFID-Antenne 105 durch eigene Leiterbahnen 103, 104 gebildet. Die RFID-Antenne 105 und das Heizorgan 17 können auf derselben Seite des Leiterkartenabschnitts 40 oder auf gegenüberliegenden Seiten angeordnet bzw. ausgebildet sein. Vorzugsweise kann die RFID-Antenne 105 derart auf dem Leiterkartenabschnitt 40 angeordnet sein, dass sie in einem durch sie eingeschlossenen Innenbereich das Heizorgan 17 in der Ebene des Leiterkartenabschnitts 40 umschließt. Die Lage und/oder Position des RFID-Chips 102, die Anzahl der RFID-Chips 102, sowie die Lage und/oder Position und/oder Ausrichtung der oder jeder RFID-Antenne 105 in Bezug auf das Heizorgan 17 kann variieren. Die Datenübertragung erfolgt optional drahtlos. Dazu kann z.B. der RFID-Chip 102 eine Antenne enthalten.

Der Leiterkartenabschnitt 40 der Verdampfereinheit 10 ist vorzugsweise aus Polyimid gebildet, wobei andere flexible Substratmaterialien ebenfalls einsetzbar sind. Besonders bevorzugt ist ein dotierter Siliziumchip als Heizorgan 17 durch Direktkontaktierung mittels Silbersintern elektrisch mit den Leiterbahnen 13, 14 verbunden. Als Heizorgan 17 können auch andere Bauteile, insbesondere im Wesentlichen aus Silizium bestehende oder Silizium oder p- oder n-dotiertes Silizium aufweisende MEMS-Bauteile (Micro-electro-mechanical-Bauteile), die flüssigkeitspermeabel ausgebildet sind, eingesetzt werden. Insbesondere können auch so genannte Folienverdampfer/Folienheizer als Heizorgan 17 verwendet werden. Der Leiterkartenabschnitt 40 sowie der Siliziumchip sind mindestens teilweise von Silikon oder Polyimid umschlossen, so dass einerseits der Siliziumchip mechanisch gehalten ist und andererseits nach außen hin, vorzugsweise definierte, Dichtflächen 21 gebildet sind. Anstelle von Silikonen können auch andere insbesondere im Film-Assisted-Moulding-Verfahren verarbeitbare Werkstoffe, die im verarbeiteten Zustand dichtend wirken, eingesetzt werden. Beispiele für weitere Werkstoffe, die mit dem Film-Assisted-Moulding-Verfahren verarbeitbar sind, sind Polyimid oder Kunststoffe.

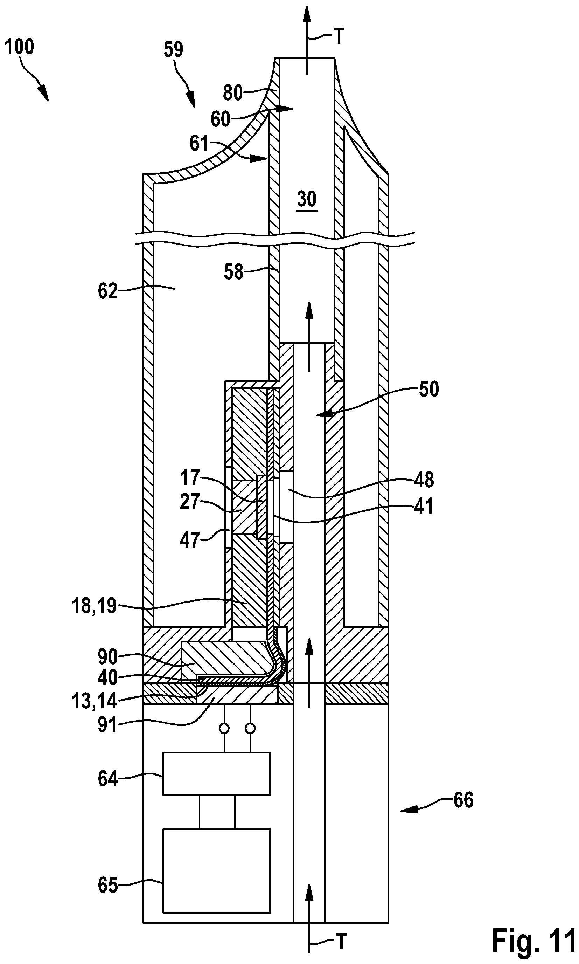

In einer bevorzugten Weiterbildung steht ein Abschnitt der in dem flexiblen Leiterkartenmaterial 11 eingebetteten Leiterbahnen 13, 14 aus der durch das Dichtmaterial 18 gebildeten Ummantelung 19 zur Bildung einer Flexkontaktierung 43 biegbar hervor. Durch Umbiegen des heraustehenden Abschnitts beispielsweise um 90° kann eine platzsparende Kontaktierungsmöglichkeit der Leiterbahnen 13, 14 erzeugt werden. Die Figur 1a) stellt die Ausführungsform dar, bei der die Flexkontaktierung 43 noch nicht umgebogen ist. Die Figur 1b) stellt die Flexkontaktierung 43 in umgebogenem Zustand dar. Wie aus den Ausführungsformen der bevorzugten Verdampfereinheiten 10 insbesondere in den Figuren 1 und 2 sowie 8 bis 11 weiter ersichtlich, ist die Heizfläche 20 auf der Oberseite O mindestens teilweise, vorzugsweise jedoch vollständig von einem Dochtorgan 27 abgedeckt. Wie bereits erwähnt, kann das Dochtorgan 27 unterschiedliche Bauformen aufweisen und aus unterschiedlichen Materialien bestehen. Optional kann das Dochtorgan 27 auch an der Unterseite angeordnet sein, sofern bei der Montage darauf geachtet wird, dass bei einer Fluidverbindung der Pfad Dochtorgan 27 - Heizorgan 17 - Luftströmungskanal 30 eingehalten wird.

Besonders bevorzugt ist die Verdampfereinheit 10 mit einem Verfahren nach einem oder mehreren der Ansprüche 1 bis 23 hergestellt. Die oder jede Verdampfereinheit 10 kann auf einer Rolle oder einem Magazin bevorratet oder als Einzelteil, Austauschteil, Ersatzteil oder dergleichen verwendet, bevorratet und eingesetzt werden. Vorzugsweise ist die Verdampfereinheit 10 jedoch Bestandteil einer Verdampferbaugruppe 44, die ihrerseits Bestandteil eines Inhalators 100 ist. Die Verdampferbaugruppe 44 umfasst eine Verdampfereinheit 10, vorzugsweise nach einem oder mehreren der Ansprüche 24 bis 30, sowie einen Adapterstecker 45, in den die Verdampfereinheit 10 einsteckbar und im Funktionszustand, also in montiertem Zustand auch eingesteckt ist, wobei die Verdampfereinheit 10 bzw. deren Ummantelung 19 zumindest teilweise mit einer Außenfläche der Ummantelung 19, also den Dichtflächen 21, mindestens teilweise dichtend an einer inneren Geometrie des Adaptersteckers 45 anliegt. Bereits durch die Montage, die ein einfaches Einschieben der Verdampfereinheit 10 in den Adapterstecker 45 beinhaltet, ergibt sich eine dichtende Einheit, bei der eine Fluidverbindung nur über den Pfad Öffnung 47 im Adapterstecker 45 - Dochtorgan 27 - Heizorgan 17 - Durchlassöffnung 41 - Öffnung 48 besteht. Mit anderen Worten kann eine Flüssigkeit im Vorratstank 62 einer Verdampferkartusche 59 nur über den vorgenannten Pfad in den Abschnitt des Luftströmungskanals 50 und weiter in den Luftströmungskanal 30 (nach vorheriger Verdampfung an oder im Heizorgan 17) gelangen.

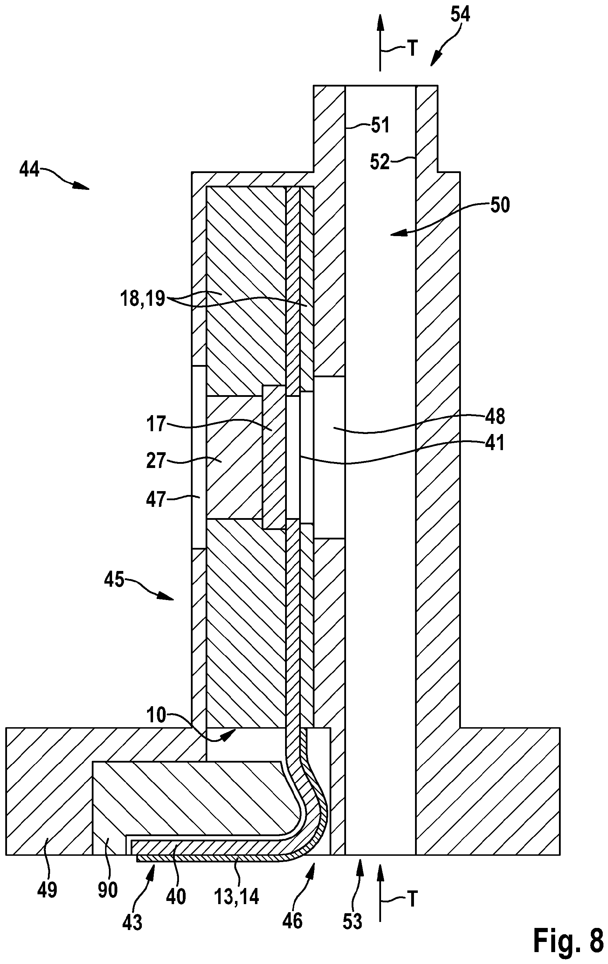

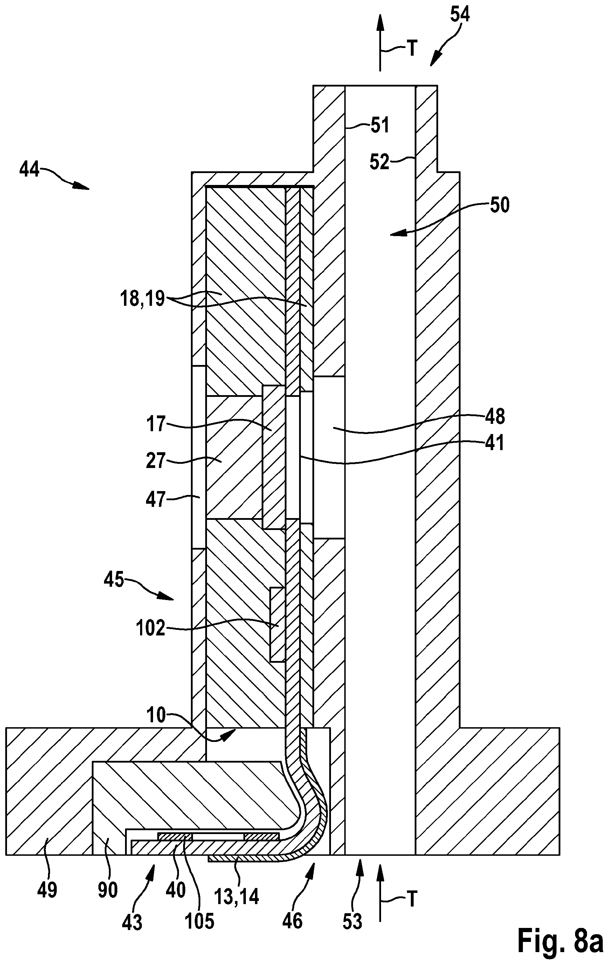

In der Figur 8 ist eine bevorzugte Ausführungsform einer Verdampferbaugruppe 44 dargestellt. In dieser Ausführungsform ist die Verdampfereinheit 10 in eine Aufnahme 46, Tasche oder dergleichen des Adaptersteckers 45 eingesteckt. Die Aufnahme 46 weist eine Öffnung 47 zur Herstellung einer Fluidverbindung zu einem Vorratstank auf. Die Aufnahme 46 weist weiterhin eine Öffnung 48 in Richtung eines Luftströmungskanals 30 auf. Zwischen diesen Öffnungen 47, 48 ist die Verdampfereinheit 10 mit ihrem Dochtorgan 27 und ihrem Heizorgan 17 platziert, so dass aus einem Vorratstank kommende Flüssigkeit zwangsläufig durch die Verdampfereinheit 10 in Richtung des Luftströmungskanals 30 strömen und durch die Verdampfereinheit 10 in Richtung des Luftströmungskanals 30 verdampft werden kann. Optional kann auch noch ein Steckereinsatz 90 als Bestandteil der Verdampferbaugruppe 44 vorgesehen sein, der zum Fixieren/Halten der Flexkontaktierung 43 in der umgebogenen Position ausgebildet und eingerichtet ist. In der Figur 8a ist eine Ausführungsform dargestellt, in der neben dem Heizorgan 17 ein RFID-Chip 102 vorgesehen ist. Ergänzend zum RFID-Chip 102 oder alternativ zu diesem kann zusätzlich mindestens ein weiterer ID-Chip oder ein anderes elektronisches Bauteil vorgesehen sein.

Die Verdampfereinheit 10 ist jedoch auch in anderer Weise form- und/oder kraftschlüssig dichtend mit dem Adapterstecker 45 verbindbar. Das Dichtmaterial 18 der Ummantelung 19 einer Verdampfereinheit 10 aus Figur 7 dichtet mit den Dichtflächen 21 die Verdampfereinheit 10 gegenüber dem Adapterstecker 45 in Figur 8 ab, so dass das Auslaufen von Flüssigkeit zwischen Verdampfereinheit 10 und Adapterstecker 45 ohne zusätzliche Dichtmittel wirksam verhindert wird. Der Adapterstecker 45 weist optional vorzugsweise einen flanschartigen Deckelabschnitt 49 auf, der z.B. als Anschlag und/oder als Deckel für einen Vorratstank ausgebildet und eingerichtet ist.

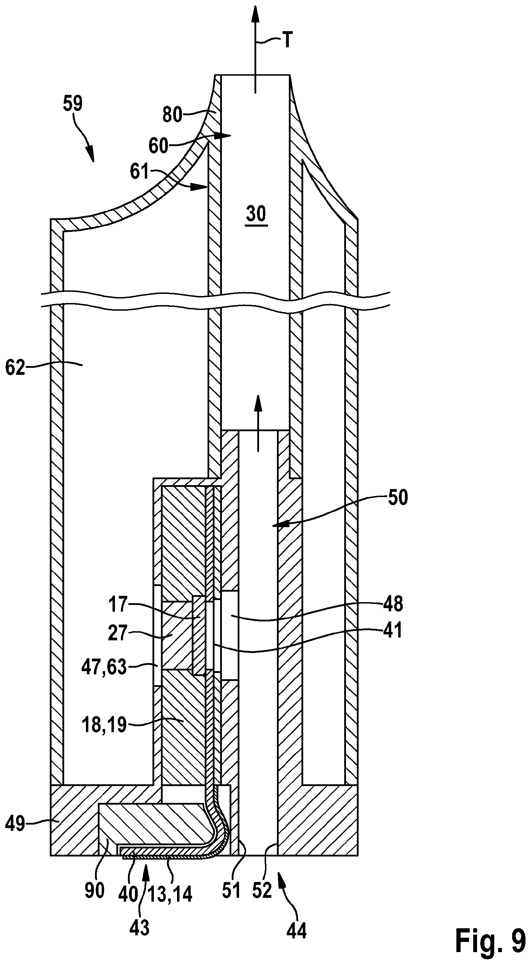

Die Verdampferbaugruppe 44 umfasst mindestens einen Abschnitt 50 eines Luftströmungskanals 30, der durch die Verdampfereinheit 10 und/oder den Adapterstecker 45 gebildet ist. In den Figuren 8 und 9 ist der Abschnitt 50 durch den Adapterstecker 45 ausgebildet, indem der Luftströmungskanal 30 durch eine Wandung 51 der Aufnahme 46 und eine Wandung 52 des Abschnittes 50 begrenzt und gebildet ist. Der Abschnitt 50 kann aber auch teilweise oder vollständig durch die Verdampfereinheit 10 gebildet sein. Wie weiter oben bereits beschrieben, kann die Verdampfereinheit 10 wie in Figur 7 gezeigt auf ihrer Unterseite stegartige Abschnitte 29 aus Dichtmaterial 28 aufweisen. Diese stegartigen Abschnitte 29 bilden alleine in einer Art "offenen Variante" einen im Umfang nur teilweise geschlossenen Kanalabschnitt und erst in montiertem Zustand mit dem Adapterstecker 45 zusammen mit der Wandung 52 einen umlaufend geschlossenen, jedoch zu beiden Stirnseiten 53, 54 hin offenen und durchgängigen Luftströmungskanal 30. In anderen Ausführungen, wie z.B. Figur 2, stellt die Verdampfereinheit 10 in einer Art "geschlossenen Variante" alleine einen Rohrabschnitt 31 zur Bildung eines Teils des Luftströmungskanals 30 zur Verfügung. Wie weiter oben bereits ausgeführt, kann der Rohrabschnitt 31 mit dem zweiten Material 32, vorzugsweise einem Dichtmaterial, an der Verdampfereinheit 10 fixiert sein. Der Rohrabschnitt 31 kann optional z.B. durch Klebung oder dergleichen fest und dauerhaft mit der Verdampfereinheit 10 verbunden sein. Die Wandung 52 dient dann nur als Dichtfläche zur Verdampfereinheit 10. Der Rohrabschnitt 31 als vorgefertigtes Luftströmungskanalelement kann aus Blech, Keramik, Silikon, Polyimid, Kunststoff oder anderen Werkstoffen gebildet sein, vorzugsweise aus einem endlosen Strangprofil. Die Querschnittsform kann variieren, wobei ein runder Querschnitt bevorzugt ist. Dieser kann aber z.B. auch halbrund, quadratisch oder rechteckig sein. Der Rohrabschnitt 31 kann insbesondere für den Fall, dass der aus Blech gefertigt ist, in seiner Formgebung, z.B. in Form von Sicken oder dergleichen, Konturveränderungen im Querschnitt aufweisen, um die Luftführung im Luftströmungskanal 30 kontrolliert zu beeinflussen.

Dieser Rohrabschnitt 31 und/oder der Abschnitt 50 des Luftströmungskanals 30 sind mit weiteren Abschnitten des Luftströmungskanals 30 vom Inhalator 100 zur Bildung eines durchgängigen Schlots im Inhalator 100 koppelbar (hierzu weiter unten). Für den Fall, dass der Rohrabschnitt 31 mit dem zweiten Dichtmaterial 32 an der Verdampfereinheit 10 fixiert ist, können aus dem zweiten Dichtmaterial 32 z.B. zwei umlaufende Dichtungskonturen 56, 57 angeformt sein - ähnlich einem O-Ring -, derart, dass die Verdampfereinheit 10 mit dem Rohrabschnitt 31 direkt dichtend in einen Abschnitt 58 des Luftströmungskanals 30 einer Verdampferkartusche 59 zur Bildung eines Schlots 60 eingesetzt ist, wobei der Innendurchmesser des Abschnitts 58 mindestens teilweise/abschnittsweise größer ist als der Außendurchmesser der Verdampfereinheit 10 inklusive angebundenem Rohrabschnitt 31 (siehe insbesondere Figur 10). Die montierte Verdampferbaugruppe 44 mit der Verdampfereinheit 10 bzw. die Verdampfereinheit 10 trennt durch die Dichtungskonturen 56, 57 den aerosolerzeugenden Abschnitt (Vorratstank - Oberseite der Verdampfereinheit) von dem aerosolführenden Abschnitt (Unterseite Verdampfereinheit - Luftströmungskanal). Es ist aber auch denkbar, dass der Innendurchmesser des Abschnitts 58 mindestens teilweise/abschnittsweise kleiner ist als der Außerdurchmesser der Verdampfereinheit 10 inklusive angebundenem Rohrabschnitt 31.

Entsprechend ist die Verdampferbaugruppe 44 bzw. die Verdampfereinheit 10 vorzugsweise Bestandteil einer Verdampferkartusche 59 (siehe insbesondere Figuren 9 und 10) als Bestandteil eines Inhalators 100. Die Verdampferkartusche 59 umfasst einen Hohlkörper 61 mit mindestens einem Abschnitt 58 eines Luftströmungskanals 30, einen Vorratstank 62 zum Bevorraten von Flüssigkeit, sowie eine Verdampferbaugruppe 44, vorzugsweise nach Anspruch 31 oder 32, wobei die Verdampferbaugruppe 44 mit dem Hohlkörper 61 und dem Vorratstank 62 derart dichtend verbunden ist, dass der Abschnitt 50 bzw. Rohrabschnitt 31 des Luftströmungskanals 30 der Verdampferbaugruppe 44 und der Abschnitt 58 des Luftströmungskanals 30 des Hohlkörpers 61 einen zum Vorratstank hin flüssigkeitsdichten, gemeinsamen Luftströmungskanal 30 bzw. Schlot 60 bilden und der Vorratstank 62 mindestens eine Zugangsöffnung 63 (z.B. in Form der Öffnung 47 in Figur 9 oder in Form der Öffnung 63 in Figur 10) zum Luftströmungskanal 30 aufweist, in deren Bereich die Verdampfereinheit 10 platziert ist. Die Verbindung zwischen dem Abschnitt 50 und dem Abschnitt 58 zur Bildung des Schlots 60 in Figur 9 kann z.B. mittels einer Presspassung erfolgen. Es ist aber auch denkbar, dass in dem Verbindungsbereich zwischen dem Abschnitt 50 und dem Abschnitt 58 ein dichtendes Element angeordnet ist, das dazu eingerichtet ist, einen flüssigkeitsdichten Übergang zwischen dem Abschnitt 50 und dem Abschnitt 58 bereitzustellen. Ein derartiges Element könnte beispielsweise aus einem Silikon, Polyimid, einem Gummi oder einem anderen geeigneten elastischen und dichtenden Werkstoff ausgebildet sein. Ein derartiges Element könnte beispielsweise in Form eines Dichtungsrings oder einer Dichtungshülse, einer dichtenden Manschette oder eines Übergangsstücks vorliegen. Das Übergangsstück könnte derart in Form eines Zylinders ausgestaltet sein und im Bereich einer ersten Stirnseite des Zylinders mit dem Abschnitt 50 dichtend eingreifen und im Bereich einer zweiten Stirnseite des Zylinders mit dem Abschnitt 58 dichtend eingreifen. In der Figur 10 ist der Abschnitt 58 in Richtung Mundstück 80 konvergierend geformt, um einen möglichst übergangsfreien Luftströmungskanal 30/Schlot 60 zu schaffen.

Bei der Ausführung gemäß Figur 9 schließt der Deckelabschnitt 49 der Verdampferbaugruppe 44 den Vorratstank 62 flüssigkeitsdichtend ab. Dadurch ist eine gegenüber der Umgebung vor Flüssigkeitsaustritt abgedichtete Verdampferkartusche 44 gebildet, die einerseits einen durchgängigen Luftströmungskanal 30 für einen mit Aerosolen versetzten Luftstrom zum Inhalieren zur Verfügung stellt, wobei der Luftströmungskanal 30 gegenüber dem unerwünschten Eindringen von Flüssigkeit aus dem Vorratstank 62 gedichtet ist, und die andererseits eine Fluidverbindung zwischen dem Vorratstank 62 und dem Luftströmungskanal 30 herstellt, derart, dass Flüssigkeit aus dem Vorratstank 62 in den Bereich der Verdampfereinheit 10 gelangt/transportierbar ist und der in oder an der Verdampfereinheit 10 aus der Flüssigkeit erzeugte Dampf an den Luftströmungskanal 30 abgebbar ist. Bei der Ausführungsform gemäß Figur 10 sind die entsprechenden Funktionalitäten ebenfalls gewährleistet. Allerdings ist der Vorratstank 62 durch einen separaten Deckel 81 abgedichtet. Die Verdampferkartusche 59 kann weiterhin ein Mundstück 80 umfassen. Es ist jedoch auch denkbar, dass die in Figur 10 gezeigte Verdampferkartusche 59 einen Adapterstecker 45 wie in Figur 8 und 9 gezeigt aufweist und die Verdampferbaugruppe 44 aus Figur 2 und 10 in dem Adapterstecker 45 montiert ist. Es ist auch denkbar, dass die in Figur 9 gezeigte Verdampferkartusche 59 einen Aufbau wie in Figur 10 mit einem separaten Deckel 81 aufweist. In beiden vorgenannten Fällen gilt das zu Figur 9 und 10 oben genannte in analoger und direkt übertragbarer Weise.

Die Erfindung betrifft des Weiteren einen Inhalator 100, ausgebildet und eingerichtet zum Inhalieren von mit Wirkstoffen und/oder Aromastoffen angereichertem Dampf/Aerosol, der mindestens eine elektronische Steuereinheit 64 und eine Energiequelle 65 umfassenden Kartuschenträger 66 sowie eine Verdampferkartusche 44 nach Anspruch 26 umfasst. Der Inhalator 100 ist als E-Zigarette dargestellt. Der Inhalator 100 kann aber ohne jede konstruktive Anpassung nur durch Auswahl der zu inhalierenden Stoffe im medizinischen und/oder therapeutischen Bereich eingesetzt werden. Optional kann der Kartuschenträger 66 einen Gegenkontakt 91 umfassen, der auf der einen Seite mit der Steuereinheit 64 und der elektrischen Energiequelle 65 verbunden ist und auf der anderen Seite mit den Leiterbahnen 13, 14 in Kontakt steht. Der Gegenkontakt 91 kann aber auch separat, z.B. als Steckteil ausgebildet sein.

Die erfindungsgemäße Verdampfereinheit 10 bzw. Verdampferbaugruppe 44 kann aber auch Bestandteil eines Inhalators 100 sein, bei dem die Verdampfereinheit 10 bzw. Verdampferbaugruppe 44 außerhalb der Verdampferkartusche 59 angeordnet ist, so dass die Verdampferbaugruppe 44 fester Bestandteil des Kartuschenträgers 66 ist, und entsprechend einen Mehrwegartikel bildet. Optional sind der Kartuschenträger 66, die Verdampferbaugruppe 44 und eine nur den Hohlkörper 61 und/oder den Vorratstank 62 umfassende Verdampferkartusche 59 in unterschiedlichen Intervallen austauschbar miteinander verbunden.

## Patentansprüche

1. Verfahren zum Herstellen von Verdampfereinheiten (10) als Bestandteil von Inhalatoren (100), umfassend die Schritte:
a) Bereitstellen eines flexiblen Leiterkartenmaterials (11) mit einer Vielzahl von Bauplätzen (12) für einzelne Verdampfereinheiten (10), wobei das flexible Leiterkartenmaterial (11) mindestens mit hinsichtlich Position und/oder Verlauf vorbestimmten Leiterbahnen (13, 14) und/oder Vorstanzungen (15, 16, 24) für jeden Bauplatz (12) wahlweise vorstrukturiert ist oder vorstrukturiert wird,
b) Bereitstellen und Platzieren mindestens eines elektrisch mit den Leiterbahnen (13, 14) wahlweise verbundenen oder verbindbaren Heizorgans (17) an dem oder jedem Bauplatz (12), und
c) mindestens teilweises Abdecken jedes Bauplatzes (12) mit einem Dichtmaterial (18) zur Bildung einer partiellen Ummantelung (19) für jede gebildete Verdampfereinheit (10),
**dadurch gekennzeichnet, dass** das Dichtmaterial (18) derart aufgebracht wird, dass die aus Dichtmaterial (18) gebildete Ummantelung (19) jedes Heizorgan (17) unter Freihaltung einer Heizfläche (20) mindestens auf einer Oberseite (O) des flexiblen Leiterkartenmaterials (11) mindestens in einem Randbereich abdeckt, und mindestens die vom Leiterkartenmaterial (11) und/oder dem Heizorgan (17) wegweisenden Außenflächen der Ummantelung (19) Dichtflächen (21) bilden.

2. Verfahren nach Anspruch 1, **dadurch gekennzeichnet, dass** im Schritt a) flexibles Leiterkartenmaterial (11) bereitgestellt wird, bei dem im Bereich jedes Bauplatzes (12) für eine einzelne Verdampfereinheit (10) mindestens ein Bauplatz (101) für einen RFID-Chip (102) ausgebildet ist, und dass das flexible Leiterkartenmaterial (11) hinsichtlich Position und/oder Verlauf vorbestimmter Leiterbahnen (103, 104) als RFID-Antenne (105) vorstrukturiert ist oder vorstrukturiert wird.

3. Verfahren nach Anspruch 2, **dadurch gekennzeichnet, dass** das flexible Leiterkartenmaterial (11) mindestens mit hinsichtlich Position und/oder Verlauf vorbestimmten Leiterbahnen und/oder Vorstanzungen für jeden Bauplatz (101) zur elektronischen Anbindung mindestens des RFID-Chips (102) wahlweise vorstrukturiert ist oder vorstrukturiert wird.

4. Verfahren nach Anspruch 2 oder 3, **dadurch gekennzeichnet, dass** in einem Schritt k), der vor, mit oder nach dem Schritt b) erfolgen kann, an jedem Bauplatz (101) ein RFID-Chip (102) bereitgestellt und platziert wird.

5. Verfahren nach Anspruch 4, **dadurch gekennzeichnet, dass** der elektrisch mit den Leiterbahnen wahlweise verbundene oder verbindbare RFID-Chip (102) bereitgestellt und platziert wird, wobei der RFID-Chip (102) an jedem Bauplatz (101) mit den die RFID-Antenne (105) bildenden Leiterbahnen (103, 104) wahlweise verbunden oder verbindbar ist.

6. Verfahren nach einem oder mehreren der Ansprüche 2 bis 5, **dadurch gekennzeichnet, dass** im Schritt c) zusätzlich zum teilweise Abdecken jedes Bauplatzes (12) für das Heizorgan (17) auch der Bauplatz (101) für den RFID-Chip (102) mit dem Dichtmaterial (18) unter Bildung von Dichtflächen abgedeckt wird.

7. Verfahren nach einem oder mehreren der Ansprüche 1 bis 6, **dadurch gekennzeichnet, dass** das Bereitstellen des flexiblen Leiterkartenmaterials (11) in Schritt a) wahlweise durch Abrollen des auf Rollen bevorrateten Leiterkartenmaterials (11) oder durch Zuführen von in Magazinen oder dergleichen bevorrateten Abschnitten des Leiterkartenmaterials (11) erfolgt.

8. Verfahren nach einem oder mehreren der Ansprüche 1 bis 7 , **dadurch gekennzeichnet, dass** im Schritt c) jeder Bauplatz (12, 101) mindestens teilweise mit Dichtmaterial (18) umschlossen wird, derart, dass zusätzlich sowohl auf der Oberseite (O) als auch auf einer der Oberseite (O) gegenüberliegenden Unterseite (U) des flexiblen Leiterkartenmaterials (11) an den Außenflächen der Ummantelung (19) Dichtflächen (21) für eine frei von zusätzlichen Dichtmitteln bestehende Verbindung zu den die Verdampfereinheit (10) umgebenden Komponenten des Inhalators (100) ausgebildet werden.

9. Verfahren nach einem oder mehreren der Ansprüche 1 bis 8 , **dadurch gekennzeichnet, dass** der Schritt b) als Schritt b₁) das Auftragen von elektrischem Kontaktierungsmaterial (25) mindestens auf Kontaktflächen (26) der Leiterbahnen (13, 14) an jedem Bauplatz (12) auf der Oberseite (O) des flexiblen Leiterkartenmaterials (11), als Schritt b₂) das Platzieren des mindestens einen Heizorgans (17) im Bereich jedes Bauplatzes (12), derart, dass eine die Oberseite (O) mit der Unterseite (U) des Leiterkartenmaterials (11) verbindende Vorstanzung (16) vollständig oder teilweise durch das mindestens eine Heizorgan (17) abgedeckt ist, und als Schritt b₃) das Ausbilden einer elektrischen Verbindung zwischen Heizorgan (17) und Leiterbahnen (13, 14) an jedem Bauplatz (12) umfasst.

10. Verfahren nach einem oder mehreren der Ansprüche 4 bis 8, **dadurch gekennzeichnet**, der Schritt k) als Schritt k₁) das Auftragen von elektrischem Kontaktierungsmaterial (25) mindestens auf Kontaktflächen der Leiterbahnen an jedem Bauplatz (101) auf der Oberseite (O) des flexiblen Leiterkartenmaterials (11), als Schritt k₂) das Platzieren des mindestens einen RFID-Chips (102) im Bereich jedes Bauplatzes (101), und als Schritt k₃) das Ausbilden einer elektrischen Verbindung zwischen RFID-Chip (102) und Leiterbahnen an jedem Bauplatz (101) umfasst.

11. Verfahren nach einem oder mehreren der Ansprüche 1 bis 10, **dadurch gekennzeichnet, dass** nach dem Schritt c) die beim Abdecken bzw. Umschließen der Bauplätze (12) mit Dichtmaterial (18) entstehenden Angussabschnitte des Dichtmaterials (18) in einem Schritt d) entfernt werden.

12. Verfahren nach einem oder mehreren der Ansprüche 7 bis 11 **dadurch gekennzeichnet, dass** nach dem Schritt c) oder d) das mit der Vielzahl fertiggestellter Verdampfereinheiten (10) versehene Leiterkartenmaterial (11) zurweiteren Verwendung in einem Schritt e) wahlweise wieder auf eine Rolle aufgerollt oder in einem Magazin oder dergleichen gesammelt wird.

13. Verfahren nach einem oder mehreren der Ansprüche 1 bis 12, **dadurch gekennzeichnet, dass** die Verdampfereinheiten (10) zur weiteren Verwendung in einem Schritt f) von dem flexiblen Leiterkartenmaterial (11) vereinzelt werden.

14. Verfahren nach einem oder mehreren der Ansprüche 1 bis 13, **dadurch gekennzeichnet, dass** in einem Schritt g) auf oder an der Oberseite (O) des flexiblen Leiterkartenmaterials (11) auf wenigstens einem Teil der freien Heizflächen (20) der Heizorgane (17) an jedem Bauplatz (12) mindestens ein Dochtorgan (27) platziert wird.

15. Verfahren nach Anspruch 14, **dadurch gekennzeichnet, dass** jedes Dochtorgan (27) zusammen mit dem Schritt b) oder nach dem Schritt b) in beliebiger Schrittfolge platziert wird.

16. Verfahren nach einem oder mehreren der Ansprüche 1 bis 15, **dadurch gekennzeichnet, dass** in einem Schritt h), vorzugsweise zusammen mit dem Schritt b₂) bzw. k₂), mindestens ein zusätzliches elektronisches Bauteil im Bereich jedes Bauplatzes (12) platziert wird.

17. Verfahren nach Anspruch 16, **dadurch gekennzeichnet, dass** vor dem Schritt h), vorzugsweise zusammen mit Schritt b₁) und k₁), Kontaktierungsmaterial (25) auf Kontaktflächen (26) der Leiterbahnen (13, 14) an jedem Bauplatz (12) auf der Oberseite (O) und/oder der Unterseite (U) des flexiblen Leiterkartenmaterials (11) aufgebracht wird, und dass nach dem Schritt h), vorzugsweise zusammen mit Schritt b₃) bzw. k₃), eine elektrische Verbindung zwischen dem oder jedem zusätzlichen elektronischen Bauteil und Leiterbahnen (13, 14) an jedem Bauplatz (12) ausgebildet wird.

18. Verfahren nach einem oder mehreren der Ansprüche 1 bis 17, **dadurch gekennzeichnet, dass** jedes Heizorgan (17) und/oder jeder RFID-Chip (102) und/oder jedes zusätzliche elektronische Bauteil elektrisch mit den Leiterbahnen (13, 14) des flexiblen Leiterkartenmaterials (11) verbunden wird durch Einsatz eines oder mehrerer Verfahren aus der Liste: Direktkontaktierung mittels Silber-Sintern, eutektisch Bonden, leitfähig Kleben, anisotrop leitfähig Kleben, KlettWelding, KlettSintering, Löten, Schweißen.

19. Verfahren nach einem oder mehreren der Ansprüche 1 bis 18, **dadurch gekennzeichnet, dass** im Schritt c) beim Abdecken bzw. Umschließen jedes Bauplatzes (12) aus und/oder mit dem Dichtmaterial (18) auf der Unterseite (U) des Leiterkartenmaterials (11) an jedem Bauplatz (12) mindestens Abschnitte (29) eines Luftströmungskanals (30) gebildet werden.

20. Verfahren nach einem oder mehreren der Ansprüche 1 bis 19, **dadurch gekennzeichnet, dass** an jedem Bauplatz (12) ein rohrförmiger, vorkonfektionierter Abschnitt (31) als Teil eines Luftströmungskanals (30) auf der Unterseite (U) des Leiterkartenmaterials (11) platziert und mittels zusätzlichem Dichtmaterial (32) umschlossen und damit fixiert wird.

21. Verfahren nach einem oder mehreren der Ansprüche 1 bis 20, **dadurch gekennzeichnet, dass** mindestens die Schritte a) bis c) nacheinander jeweils an einer eigenen oder der gleichen Fertigungsstation (34 bis 39) einer Fertigungslinie (33) ausgeführt werden, derart, dass bei jedem Schritt eine Vielzahl von Bauplätzen (12) zeitgleich bearbeitet wird.

22. Verfahren nach Anspruch 21, **dadurch gekennzeichnet, dass** der gesamte Herstellungsprozess der Verdampfereinheiten (10) in einer gemeinsamen Fertigungslinie (33) mit mehreren Fertigungsstationen (34 bis 39) erfolgt.

23. Verfahren nach Anspruch 21 oder 22, **dadurch gekennzeichnet, dass** das flexible und vorzugsweise aus Polyimid bestehende, vorstrukturierte Leiterkartenmaterial (11) als Endlosstrang von einer Vorratsrolle abgewickelt und kontinuierlich oder intermittierend durch die mehrere Fertigungsstationen (34 bis 39) umfassende Fertigungslinie (33) transportiert wird.

24. Verdampfereinheit (10) zur Verwendung als Bestandteil eines Inhalators (100), umfassend einen Leiterkartenabschnitt (40) aus flexiblem Leiterkartenmaterial (11), wobei der Leiterkartenabschnitt (40) mindestens zwei Leiterbahnen (13, 14) und eine Durchlassöffnung (41) umfasst, die eine Oberseite (O) des Leiterkartenabschnitts (40) mit einer Unterseite (U) des Leiterkartenabschnitts (40) verbindet, sowie mindestens ein Heizorgan (17), das die Durchlassöffnung (41) von der Oberseite (O) her vollständig oder teilweise abdeckt und elektrisch leitend mit den Leiterbahnen (13, 14) verbunden ist, **dadurch gekennzeichnet, dass** sie weiterhin eine aus Dichtmaterial (18) bestehende Ummantelung (19) umfasst, die den Leiterkartenabschnitt (40) und das Heizorgan (17) unter Freihaltung einer Heizfläche (20) mindestens teilweise abdeckt und nach außen hin mit Dichtflächen (21) versehen ist.

25. Verdampfereinheit nach Anspruch 24, **dadurch gekennzeichnet, dass** der Leiterkartenabschnitt (40) mindestens einen RFID-Chip (102) und mindestens eine RFID-Antenne (105) umfasst.

26. Verdampfereinheit (10) nach Anspruch 24 oder 25, **dadurch gekennzeichnet, dass** der Leiterkartenabschnitt (40) aus Polyimid gebildet ist, ein dotierter Siliziumchip als Heizorgan (17) durch Direktkontaktierung mittels Silbersintern elektrisch mit den Leiterbahnen (13, 14) verbunden ist, und der Leiterkartenabschnitt (40) sowie der Siliziumchip mindestens teilweise von Silikon oder Polyimid umschlossen sind, so dass einerseits der Siliziumchip mechanisch gehalten ist und andererseits nach außen hin, vorzugsweise definierte, Dichtflächen (21) gebildet sind.

27. Verdampfereinheit (10) nach einem oder mehreren der Ansprüche 24 bis 26, **dadurch gekennzeichnet, dass** die in dem flexiblen Leiterkartenmaterial (11) eingebetteten Leiterbahnen (13, 14) aus der durch das Dichtmaterial (18) gebildeten Ummantelung (19) zur Bildung einer Flexkontaktierung (43) biegbar hervorstehen.

28. Verdampfereinheit (10) nach einem oder mehreren der Ansprüche 24 bis 27, **dadurch gekennzeichnet, dass** die Heizfläche (20) des Heizorgans (17) auf der Oberseite mindestens teilweise von einem Dochtorgan (27) abgedeckt ist.

29. Verdampfereinheit (10) nach einem oder mehreren der Ansprüche 24 bis 28, **dadurch gekennzeichnet, dass** auf der Unterseite (U) des Leiterkartenabschnitts (40) mindestens ein Rohrabschnitt (31) als Abschnitt eines Luftströmungskanals (30) ausgebildet und/oder angeordnet ist, wobei der Rohrabschnitt (31) eine zum Heizorgan (17) gerichtete Öffnung (55) aufweist.

30. Verdampfereinheit (10) nach einem oder mehreren der Ansprüche 24 bis 29, **dadurch gekennzeichnet, dass** sie mit einem Verfahren nach einem oder mehreren der Ansprüche 1 bis 23 hergestellt ist.

31. Verdampferbaugruppe (44) als Bestandteil eines Inhalators (100), **gekennzeichnet durch** eine Verdampfereinheit (10) nach einem oder mehreren der Ansprüche 24 bis 30 sowie einen Adapterstecker (45), in den die Verdampfereinheit (10) eingesteckt ist, wobei die Verdampfereinheit (10) bzw. deren Ummantelung (19) zumindest teilweise mit einer Außenfläche der Ummantelung (19) dichtend an einer inneren Geometrie des Adaptersteckers (45) anliegt.

32. Verdampferbaugruppe (44) nach Anspruch 31, **dadurch gekennzeichnet, dass** sie mindestens einen Abschnitt (31, 50) eines Luftströmungskanals (30) umfasst, der durch die Verdampfereinheit (10) und/oder den Adapterstecker (45) gebildet ist.

33. Verdampferkartusche (59) als Bestandteil eines Inhalators (100), **gekennzeichnet durch** einen Hohlkörper (61) mit mindestens einem Abschnitt (58) eines Luftströmungskanals (30), einen Vorratstank (62) zum Bevorraten von Flüssigkeit, sowie eine Verdampferbaugruppe (44) nach Anspruch 31 oder 32, wobei die Verdampferbaugruppe (44) mit dem Hohlkörper (61) und dem Vorratstank (62) derart dichtend verbunden ist, dass der Abschnitt (50) des Luftströmungskanals (30) der Verdampferbaugruppe (44) und der Abschnitt (58) des Luftströmungskanals (30) des Hohlkörpers (61) einen gemeinsamen Luftströmungskanal (30) bilden und der Vorratstank (62) mindestens eine Zugangsöffnung (63) zum Luftströmungskanal (30) aufweist, in der die Verdampfereinheit (10) platziert ist.

34. Inhalator (100), ausgebildet und eingerichtet zum Inhalieren von mit Wirkstoffen und/oder Aromastoffen angereichertem Dampf/Aerosol, **gekennzeichnet durch** einen mindestens eine elektronische Steuereinheit (64) und eine elektrische Energiequelle (65) umfassenden Kartuschenträger (66) sowie eine Verdampferkartusche (59) nach Anspruch 33.

## Claims

1. Method for producing vaporizer units (10) as a component of inhalers (100), comprising the steps:
a) providing a flexible circuit board material (11) with a plurality of sites (12) for individual vaporizer units (10), wherein the flexible circuit board material (11) is optionally pre-structured at least with conductor paths (13, 14) and/or pre-punched areas (15, 16, 24) predetermined in terms of position and/or course for each site (12),
b) providing and placing at least one heating element (17) which is optionally connected or can optionally be connected electrically to the conductor paths (13, 14) at the or each site (12), and
c) at least partial covering of each site (12) with a sealing material (18) for the formation of a partial sheathing (19) for each formed vaporizer unit (10), **characterised in that** the sealing material (18) is applied in such a manner that the sheathing (19) formed from sealing material (18) covers each heating element (17) while keeping open a heating surface (20) at least on an upper side (O) of the flexible circuit board material (11) at least in an edge region, and at least the outer surfaces of the sheathing (19) pointing away from the circuit board material (11) and/or the heating element (17) form sealing surfaces (21).

2. Method according to claim 1, **characterised in that** in step a) flexible circuit board material (11) is provided, in the case of which at least one site (101) for an RFID chip (102) is formed in the region of each site (12) for an individual vaporizer unit (10), and that the flexible circuit board (11) is pre-structured in terms of position and/or course of predetermined conductor paths (103, 104) as an RFID antenna (105).

3. Method according to claim 2, **characterised in that** the flexible circuit board material (11) is optionally pre-structured at least with conductor paths and/or pre-punched areas predetermined in terms of position and/or course for each site (101) for electronic connection of at least the RFID chip (102).

4. Method according to claim 2 or 3, **characterised in that** in a step k), which can be performed before, with or after step b), an RFID chip (102) is provided and placed at each site (101).

5. Method according to claim 4, **characterised in that** the RFID chip (102) which is optionally connected or can optionally be connected electrically to the conductor paths is provided and placed, wherein the RFID chip (102) is optionally connected or can optionally be connected at each site (101) to the conductor paths (103, 104) which form the RFID antenna (105).

6. Method according to one or more of claims 2 to 5, **characterised in that** in step c) the site (101) for the RFID chip (102) is covered with the sealing material (18) while forming sealing surfaces additionally for partial covering of each site (12) for the heating element (17).

7. Method according to one or more of claims 1 to 6, **characterised in that** the provision of the flexible circuit board material (11) is performed in step a) optionally by rolling out the circuit board material (11) stored on rolls or by supplying portions of the circuit board material (11) stored in magazines or the like.

8. Method according to one or more of claims 1 to 7, **characterised in that** in step c) each site (12, 101) is enclosed at least partially with sealing material (18) such that sealing surfaces (21) for an existing connection free of additional sealants to the components of the inhaler (100) surrounding the vaporizer unit (10) are formed additionally both on the upper side (O) and on a lower side (U), opposite the upper side (O), of the flexible circuit board material (11) on the outer surfaces of the sheathing (19).

9. Method according to one or more of claims 1 to 8, **characterised in that** step b) comprises as step b₁) the application of electric contacting material (25) at least on contact surfaces (26) of the conductor paths (13, 14) at each site (12) on the upper side (O) of the flexible circuit board material (11), as step b₂) the placing of the at least one heating element (17) in the region of each site (12) such that a prepunched area (16) which connects the upper side (O) to the lower side (U) of the circuit board material (11) is fully or partially covered by the at least one heating element (17), and as step b₃) the formation of an electric connection between heating element (17) and conductor paths (13, 14) at each site (12).

10. Method according to one or more of claims 4 to 8, **characterised in that** step k) comprises as step k₁) the application of electric contacting material (25) at least on contact surfaces of the conductor paths at each site (101) on the upper side (O) of the flexible circuit board material (11), as step k₂) the placing of the at least one RFID chip (102) in the region of each site (101), and as step k₃) the formation of an electric connection between RFID chip (102) and conductor paths at each site (101).

11. Method according to one or more of claims 1 to 10, **characterised in that** after step c) the sprue portions of the sealing material (18) generated when covering or enclosing the sites (12) with sealing material (18) are removed in a step d).

12. Method according to one or more of claims 7 to 11, **characterised in that** after step c) or d) the circuit board material (11) provided with the plurality of finished vaporizer units (10) is optionally rolled up again onto a roll or collected in a magazine or the like for further use in a step e).

13. Method according to one or more of claims 1 to 12, **characterised in that** the vaporizer units (10) are isolated from the flexible circuit board material (11) for further use in a step f).

14. Method according to one or more of claims 1 to 13, **characterised in that** in a step g) at least one wick element (27) is placed on or at the upper side (O) of the flexible circuit board material (11) on at least a part of the free heating surfaces (20) of the heating elements (17) at each site (12).

15. Method according to claim 14, **characterised in that** each wick element (27) is placed in any desired sequence of steps together with step b) or after step b).

16. Method according to one or more of claims 1 to 15, **characterised in that** in a step h), preferably together with step b₂) or k₂), at least one additional electronic component is placed in the region of each site (12).

17. Method according to claim 16, **characterised in that** prior to step h), preferably together with step b₁) and k₁), contacting material (25) is applied on contact surfaces (26) of the conductor paths (13, 14) at each site (12) on the upper side (O) and/or the lower side (U) of the flexible circuit board material (11), and that after step h), preferably together with step b₃) or k₃), an electric connection is formed between the or each additional electronic component and conductor paths (13, 14) at each site (12).

18. Method according to one or more of claims 1 to 17, **characterised in that** each heating element (17) and/or each RFID chip (102) and/or each additional electronic component is connected electrically to the conductor paths (13, 14) of the flexible circuit board material (11) as a result of use of one or more methods from the list: direct contacting by means of silver sintering, eutectic bonding, conductive gluing, anisotropically conductive gluing, KlettWelding, KlettSintering, soldering, welding.

19. Method according to one or more of claims 1 to 18, **characterised in that** in step c) at least portions (29) of an air flow channel (30) are formed during covering or enclosing of each site (12) from and/or with the sealing material (18) on the lower side (U) of the circuit board material (11) at each site (12).

20. Method according to one or more of claims 1 to 19, **characterised in that** at each site (12) a tubular, prefabricated portion (31) is placed as part of an air flow channel (30) on the lower side (U) of the circuit board material (11) and enclosed and thus fixed by means of additional sealing material (32).

21. Method according to one or more of claims 1 to 20, **characterised in that** at least steps a) to c) are executed consecutively in each case at a separate or the same production station (34 to 39) of a production line (33) in such a manner that a plurality of sites (12) are processed at the same time during each step.

22. Method according to claim 21, **characterised in that** the entire production process of the vaporizer units (10) is performed in a joint production line (33) with several production stations (34 to 39).

23. Method according to claim 21 or 22, **characterised in that** the flexible, pre-structured circuit board material (11) preferably composed of polyimide is unwound as a continuous strand from a supply roll and transported continuously or intermittently through the production line (33) comprising several production stations (34 to 39).

24. Vaporizer unit (10) for use as a component of an inhaler (100), comprising a circuit board portion (40) composed of flexible circuit board material (11), wherein the circuit board portion (40) comprises at least two conductor paths (13, 14) and a passage opening (41) which connects an upper side (O) of the circuit board portion (40) to a lower side (U) of the circuit board portion (40), as well as at least one heating element (17) which covers the passage opening (41) fully or partially from the upper from the upper side (O) and is connected in an electrically conductive manner to the conductor paths (13, 14), **characterised in that** it further comprises a sheathing (19) which is composed of sealing material (18) which at least partially covers the circuit board portion (40) and the heating element (17) while keeping open a heating surface (20) and is provided towards the outside with sealing surfaces (21).

25. Vaporizer unit according to claim 24, **characterised in that** the circuit board portion (40) comprises at least one RFID chip (102) and at least one RFID antenna (105).

26. Vaporizer unit according to claim 24 or 25, **characterised in that** the circuit board portion (40) is formed from polyimide, a doped silicon chip as a heating element (17) is connected by direct contacting by means of silver sintering electrically to the conductor paths (13, 14), and the circuit board portion (40) as well as the silicon chip are enclosed at least partially by silicon or polyimide so that on one hand the silicon chip is retained mechanically and on the other hand towards the outside preferably defined sealing surfaces (21) are formed.

27. Vaporizer unit (10) according to one or more of claims 24 to 26, **characterised in that** the conductor paths (13, 14) embedded in the flexible circuit board material (11) project in a bendable manner out of the sheathing (19) formed by the sealing material (18) for the formation of a flex contacting (43).

28. Vaporizer unit (10) according to one or more of claims 24 to 27, **characterised in that** the heating surface (20) of the heating element (17) is covered on the upper side at least partially by a wick element (27).

29. Vaporizer unit (10) according to one or more of claims 24 to 28, **characterised in that** at least one tube portion (31) is formed and/or arranged as a portion of an air flow channel (30) on the lower side (U) of the circuit board portion (40), wherein the tube portion (31) has an opening (55) directed towards the heating element (17).

30. Vaporizer unit (10) according to one or more of claims 24 to 29, **characterised in that** it is produced with a method according to one or more of claims 1 to 23.

31. Vaporizer assembly (44) as a component of an inhaler (100), **characterised by** a vaporizer unit (10) according to one or more of claims 24 to 30 as well as an adapter plug (45), into which the vaporizer unit (10) is plugged, wherein the vaporizer unit (10) or its sheathing (19) bears at least partially with an outer surface of the sheathing (19) in a sealing manner against an inner geometry of the adapter plug (45).

32. Vaporizer assembly (44) according to claim 31, **characterised in that** it comprises at least one portion (31, 50) of an air flow channel (30) which is formed by the vaporizer unit (10) and/or the adapter plug (45).

33. Vaporizer cartridge (59) as a component of an inhaler (100), **characterised by** a hollow body (61) with at least one portion (58) of an air flow channel (30), a supply tank (62) for storing liquid, as well as a vaporizer assembly (44) according to claim 31 or 32, wherein the vaporizer assembly (44) is connected to the hollow body (61) and the supply tank (62) in a sealing manner such that the portion (50) of the air flow channel (30) of the vaporizer assembly (44) and the portion (58) of the air flow channel (30) of the hollow body (61) form a joint air flow channel (30) and the supply tank (62) has at least one access opening (63) to the air flow channel (30) in which the vaporizer unit (10) is placed.

34. Inhaler (100), formed and configured for inhaling vapour/aerosol enriched with active ingredients and/or flavourings, **characterised by** a cartridge carrier (66) comprising at least one electronic control unit (64) and an electric energy source (65) as well as a vaporizer cartridge (59) according to claim 33.

## Revendications

1. Procédé de fabrication d'ensembles de vaporisation (10) comme partie constituante d'inhalateurs (100), comprenant les étapes consistant à :
a) fournir un matériau de cartes de circuits imprimés (11) souple comportant une pluralité de sites d'implantation (12) pour des ensembles de vaporisation (10) individuels, le matériau de cartes de circuits imprimés (11) souple facultativement peut être préstructuré ou est préstructuré pour chaque site d'implantation (12) au moins avec des pistes conductrices (13, 14) et/ou des découpes préalables (15, 16, 24) prédéfinies en termes de position et/ou de parcours,
b) fournir et mettre en place au moins un élément chauffant (17) facultativement connecté ou pouvant être connecté électriquement aux pistes conductrices (13, 14) au niveau du ou de chaque site d'implantation (12), et
c) recouvrir au moins en partie chaque site d'implantation (12) avec un matériau d'étanchéité (18) de manière à former un enrobage partiel (19) pour chaque ensemble de vaporisation (10) réalisé,
**caractérisé en ce que** le matériau d'étanchéité (18) est appliqué de telle sorte que l'enrobage (19) constitué du matériau d'étanchéité (18) recouvre chaque élément chauffant (17) en maintenant une surface chauffante (20) dégagée au moins sur une face supérieure (O) du matériau de cartes de circuits imprimés (11) souple, au moins dans une zone marginale, et qu'au moins les surfaces extérieures de l'enrobage (19) dirigées de manière éloignée du matériau de cartes de circuits imprimés (11) et/ou de l'élément chauffant (17) forment des surfaces d'étanchéité (21).

2. Procédé selon la revendication 1, **caractérisé en ce qu'**à l'étape a), le matériau de cartes de circuits imprimés (11) souple est fourni, de telle sorte que dans la zone de chaque site d'implantation (12), au moins un site d'implantation (101) est formé pour une puce RFID (102) pour un ensemble de vaporisation (10) individuel, et **en ce que** le matériau de cartes de circuits imprimés (11) souple peut être préstructuré ou est préstructuré sous la forme d'une antenne RFID (105) avec des pistes conductrices prédéfinies (103, 104) en termes de position et/ou de parcours.

3. Procédé selon la revendication 2, **caractérisé en ce que** le matériau de cartes de circuits imprimés (11) souple facultativement peut être préstructuré ou est préstructuré au moins avec des pistes conductrices et/ou des découpes préalables prédéfinies en termes de position et/ou de parcours pour chaque site d'implantation (101) pour connecter électroniquement au moins la puce RFID (102).

4. Procédé selon la revendication 2 ou 3, **caractérisé en ce qu'**une puce RFID (102) est fournie et mise en place sur chaque site d'implantation (101) lors d'une étape k) qui peut être effectuée avant, pendant ou après l'étape b).

5. Procédé selon la revendication 4, **caractérisé en ce que** la puce RFID (102) facultativement connectée ou pouvant être connectée électriquement aux pistes conductrices est fournie et mise en place, la puce RFID (102) facultativement étant connectée ou pouvant être connectée aux pistes conductrices (103, 104) formant l'antenne RFID (105) sur chaque site d'implantation (101).

6. Procédé selon une ou plusieurs des revendications 2 à 5, **caractérisé en ce que** lors de l'étape c), en plus du recouvrement partiel de chaque site d'implantation (12) pour l'élément chauffant (17), le site d'implantation (101) pour la puce RFID (102) est également recouvert du matériau d'étanchéité (18) avec formation de surfaces d'étanchéité.

7. Procédé selon une ou plusieurs des revendications 1 à 6, **caractérisé en ce que** la fourniture du matériau de cartes de circuits imprimés (11) souple à l'étape a) survient facultativement en déroulant le matériau de cartes de circuits imprimés (11) stocké sur des rouleaux ou en alimentant des sections du matériau de cartes de circuits imprimés (11) stockées dans des magasins ou similaires.

8. Procédé selon une ou plusieurs des revendications 1 à 7 , **caractérisé en ce que** lors de l'étape c), chaque site d'implantation (12, 101) est au moins partiellement entouré d'un matériau d'étanchéité (18), de telle sorte qu'en outre des surfaces d'étanchéité (21) sont formées sur les surfaces extérieures de l'enrobage (19), à la fois sur la face supérieure (O) et sur une face inférieure (U) du matériau de cartes de circuits imprimés (11) souple opposée à la face supérieure (O), pour une connexion sans agent d'étanchéité supplémentaire avec les composants de l'inhalateur (100) entourant l'ensemble de vaporisation (10).

9. Procédé selon une ou plusieurs des revendications 1 à 8 , **caractérisé en ce que** l'étape b) comprend, en tant qu'étape b₁), l'application d'un matériau de contact électrique (25) au moins sur des surfaces de contact (26) des pistes conductrices (13, 14) au niveau de chaque site d'implantation (12) sur la face supérieure (O) du matériau de cartes de circuits imprimés (11) souple, en tant qu'étape b₂), la mise en place dudit au moins un élément chauffant (17) dans la zone de chaque site d'implantation (12) de telle sorte qu'une découpe préalable (16) reliant la face supérieure (O) à la face inférieure (U) du matériau de cartes de circuits imprimés (11) soit entièrement ou partiellement recouverte par ledit au moins un élément chauffant (17), et en tant qu'étape b₃), la formation d'une connexion électrique entre l'élément chauffant (17) et les pistes conductrices (13, 14) au niveau de chaque site d'implantation (12).

10. Procédé selon une ou plusieurs des revendications 4 à 8, **caractérisé en ce que** l'étape k) comprend, en tant qu'étape k₁), l'application d'un matériau de contact électrique (25) au moins sur des surfaces de contact des pistes conductrices sur chaque site d'implantation (101) sur la face supérieure (O) du matériau de cartes de circuits imprimés (11) souple, en tant qu'étape k₂), la mise en place de ladite au moins une puce RFID (102) dans la zone de chaque site d'implantation (101), et en tant qu'étape k₃), la formation d'une connexion électrique entre la puce RFID (102) et les pistes conductrices au niveau de chaque site d'implantation (101).

11. Procédé selon une ou plusieurs des revendications 1 à 10, **caractérisé en ce qu'**après l'étape c), les sections de carotte du matériau d'étanchéité (18) qui se forment lors du recouvrement ou de l'enrobage des sites d'implantation (12) avec du matériau d'étanchéité (18) sont enlevées dans une étape d).

12. Procédé selon une ou plusieurs des revendications 7 à 11, **caractérisé en ce qu'**après l'étape c) ou d), le matériau de cartes de circuits imprimés (11) muni de la pluralité d'ensembles de vaporisation (10) finis est facultativement enroulé sur un rouleau ou collecté dans un magasin ou similaire pour une utilisation ultérieure dans une étape e).

13. Procédé selon une ou plusieurs des revendications 1 à 12, **caractérisé en ce que** les ensembles de vaporisation (10) sont séparés du matériau de cartes de circuits imprimés (11) souple pour une utilisation ultérieure dans une étape f).

14. Procédé selon une ou plusieurs des revendications 1 à 13, **caractérisé en ce que** lors d'une étape g), au moins un organe de mèche (27) est mis en place sur ou au niveau de la face supérieure (O) du matériau de cartes de circuits imprimés (11) souple sur au moins une partie des surfaces chauffantes (20) dégagées des éléments chauffants (17) sur chaque site d'implantation (12).

15. Procédé selon la revendication 14, **caractérisé en ce que** chaque organe de mèche (27) est mis en place pendant l'étape b) ou après l'étape b) dans une séquence d'étapes quelconque.

16. Procédé selon une ou plusieurs des revendications 1 à 15, **caractérisé en ce que** lors d'une étape h), de préférence conjointement avec l'étape b₂) ou k₂), au moins un composant électronique supplémentaire est mis en place dans la zone de chaque site d'implantation (12).

17. Procédé selon la revendication 16, **caractérisé en ce qu'**avant l'étape h), de préférence conjointement avec l'étape b₁) et k₁), du matériau de contact (25) est appliqué sur des surfaces de contact (26) des pistes conductrices (13, 14) sur chaque site d'implantation (12) sur la face supérieure (O) et/ou la face inférieure (U) du matériau de cartes de circuits imprimés (11) souple, et **en ce qu'**après l'étape h), de préférence conjointement avec l'étape b₃) ou k₃), une connexion électrique est formée entre le ou chaque composant électronique supplémentaire et les pistes conductrices (13, 14) sur chaque site d'implantation (12).

18. Procédé selon une ou plusieurs des revendications 1 à 17, **caractérisé en ce que** chaque élément chauffant (17) et/ou chaque puce RFID (102) et/ou chaque composant électronique supplémentaire est connecté électriquement aux pistes conductrices (13, 14) du matériau de cartes de circuits imprimés (11) souple en utilisant un ou plusieurs procédés de la liste suivante : contact direct par frittage argenté, collage eutectique, collage conducteur, collage conducteur anisotrope, soudure, frittage, brasage, soudage.

19. Procédé selon une ou plusieurs des revendications 1 à 18, **caractérisé en ce qu'**à l'étape c), au moins des sections (29) d'un canal d'écoulement d'air (30) sont formées sur la face inférieure (U) du matériau de cartes de circuits imprimés (11) sur chaque site d'implantation (12) lors du recouvrement ou de l'enrobage de chaque site d'implantation (12) à partir de et/ou avec le matériau d'étanchéité (18).

20. Procédé selon une ou plusieurs des revendications 1 à 19, **caractérisé en ce qu'**au niveau de chaque site d'implantation (12), une section tubulaire préfabriquée (31) en tant que partie d'un canal d'écoulement d'air (30) est mise en place sur la face inférieure (U) du matériau de cartes de circuits imprimés (11) et est entourée et ainsi fixée au moyen d'un matériau d'étanchéité supplémentaire (32).

21. Procédé selon une ou plusieurs des revendications 1 à 20, **caractérisé en ce qu'**au moins les étapes a) à c) sont exécutées successivement respectivement sur une propre ou sur la même station de production (34 à 39) d'une ligne de production (33), de telle sorte qu'à chaque étape, une pluralité de sites d'implantation (12) soient traités simultanément.

22. Procédé selon la revendication 21, **caractérisé en ce que** l'ensemble du processus de fabrication des ensembles de vaporisation (10) s'effectue dans une ligne de production commune (33) avec plusieurs stations de production (34 à 39).

23. Procédé selon la revendication 21 ou 22, **caractérisé en ce que** le matériau de cartes de circuits imprimés préstructuré (11) souple et de préférence constitué de polyimide est déroulé sous la forme d'un cordon continu depuis un rouleau de réserve et est transporté en continu ou par intermittence à travers la ligne de production (33) comprenant plusieurs postes de production (34 à 39).

24. Ensemble de vaporisation (10) destiné à être utilisé comme partie constituante d'un inhalateur (100), comprenant une section de cartes de circuits imprimés (40) en matériau de cartes de circuits imprimés (11) souple, la section de cartes de circuits imprimés (40) comprenant au moins deux pistes conductrices (13, 14) et une ouverture de passage (41) qui relie une face supérieure (O) de la section de carte de circuits imprimés (40) à une face inférieure (U) de la section de cartes de circuits imprimés (40), ainsi qu'au moins un élément chauffant (17) qui recouvre complètement ou partiellement l'ouverture de passage (41) à partir de la face supérieure (O) et qui est relié de manière électriquement conductrice aux pistes conductrices (13, 14), **caractérisé en ce qu'**il comprend en outre un enrobage (19) en matériau d'étanchéité (18) qui recouvre au moins partiellement la section de cartes de circuits imprimés (40) et l'élément chauffant (17) tout en maintenant une surface de chauffage (20) dégagée et qui est pourvue de surfaces d'étanchéité (21) vers l'extérieur.

25. Ensemble de vaporisation selon la revendication 24, **caractérisé en ce que** la section de cartes de circuits imprimés (40) comprend au moins une puce RFID (102) et au moins une antenne RFID (105).

26. Ensemble de vaporisation (10) selon la revendication 24 ou 25, **caractérisé en ce que** la section de cartes de circuits imprimés (40) est formée de polyimide, une puce de silicium dopée en tant qu'élément chauffant (17) est connectée électriquement aux pistes conductrices (13, 14) par contact direct au moyen d'un frittage à l'argent, et la section de cartes de circuits imprimés (40) ainsi que la puce de silicium sont au moins partiellement entourées de silicone ou de polyimide, de sorte que, d'une part, la puce de silicium soit maintenue mécaniquement et que, d'autre part, des surfaces d'étanchéité (21) de préférence définies, soient formées vers l'extérieur.

27. Ensemble de vaporisation (10) selon une ou plusieurs des revendications 24 à 26, **caractérisé en ce que** les pistes conductrices (13, 14) noyées dans le matériau de cartes de circuits imprimés (11) souple dépassent de manière flexible de l'enrobage (19) formé par le matériau d'étanchéité (18) pour former un contact flexible (43).

28. Ensemble de vaporisation (10) selon une ou plusieurs des revendications 24 à 27, **caractérisé en ce que** la surface chauffante (20) de l'élément chauffant (17) est recouverte au moins partiellement sur la face supérieure par un organe de mèche (27).

29. Ensemble de vaporisation (10) selon une ou plusieurs des revendications 24 à 28, **caractérisé en ce qu'**au moins une section de tube (31) est réalisée et/ou agencée en tant que section d'un canal d'écoulement d'air (30) sur la face inférieure (U) de la section de cartes de circuits imprimés (40), la section de tube (31) comprenant une ouverture (55) orientée vers l'élément chauffant (17).

30. Ensemble de vaporisation (10) selon une ou plusieurs des revendications 24 à 29, **caractérisé en ce qu'**il est fabriqué par un procédé selon une ou plusieurs des revendications 1 à 23.

31. Module de vaporisation (44) comme partie constituante d'un inhalateur (100), **caractérisé par** un ensemble de vaporisation (10) selon une ou plusieurs des revendications 24 à 30, ainsi qu'un connecteur adaptateur (45) dans lequel l'ensemble de vaporisation (10) est inséré, l'ensemble de vaporisation (10) ou son enrobage (19) reposant au moins partiellement avec une surface extérieure de l'enrobage (19) de manière étanche sur une géométrie intérieure du connecteur adaptateur (45).

32. Module de vaporisation (44) selon la revendication 31, **caractérisé en ce qu'**il comprend au moins une section (31, 50) d'un canal d'écoulement d'air (30) formé par l'ensemble de vaporisation (10) et/ou le connecteur adaptateur (45).

33. Cartouche de vaporisation (59) comme partie constituante d'un inhalateur (100), **caractérisée par** un corps creux (61) avec au moins une section (58) d'un canal d'écoulement d'air (30), un réservoir de stockage (62) pour stocker du liquide, ainsi qu'un module de vaporisation (44) selon la revendication 31 ou 32, le module de vaporisation (44) étant relié de manière étanche au corps creux (61) et au réservoir de stockage (62) de telle sorte que la section (50) du canal d'écoulement d'air (30) du module de vaporisation (44) et la section (58) du canal d'écoulement d'air (30) du corps creux (61) forment un canal d'écoulement d'air (30) commun et le réservoir de stockage (62) comprend au moins une ouverture d'accès (63) au canal d'écoulement d'air (30), dans laquelle l'ensemble de vaporisation (10) est placé.

34. Inhalateur (100), formé et équipé pour l'inhalation de vapeur/aérosol enrichi en substances actives et/ou en arômes, **caractérisé par** un support de cartouche (66) comprenant au moins une unité de commande électronique (64) et une source d'énergie électrique (65) ainsi qu'une cartouche de vaporisation (59) selon la revendication 33.
